# EUROPÄISCHE PATENTSCHRIFT

(11) **EP 2 029 580 B1**
(45) Veröffentlichungstag und Bekanntmachung des Hinweises auf die Patenterteilung: **16.06.2010**
(21) Anmeldenummer: 07722436.8
(22) Anmeldetag: 14.05.2007
(51) Int. Cl.: C07D 403/06, A61K 31/404, A61P 35/00

(54) **NEUE INDOL-PYRROL-DERIVATE ZUR BEHANDLUNG PROLIFERATIVER UND INFLAMMATORISCHER KRANKHEITEN**
NOVEL INDOL-PYRROL DERIVATIVES FOR THE TREATMENT OF PROLIFERATIVE AND INFLAMMATORY DISEASES
NOUVEAUX DÉRIVÉS D'INDOLE-PYRROLE POUR LE TRAITEMENT DE MALADIES PROLIFÉRATIVES ET INFLAMMATOIRES

(30) Priorität: 24.05.2006 DE 102006024834
(43) Veröffentlichungstag der Anmeldung: 04.03.2009
(73) Patentinhaber: ScheBo Biotech AG, 35394 Giessen (DE)
(72) Erfinder: SCHEEFERS, Hans, 35435 Wettenberg-Wissmar (DE)
(74) Vertreter: Jungblut, Bernhard Jakob
(86) Internationale Anmeldenummer: PCT/DE2007/000888
(87) Internationale Veröffentlichungsnummer: WO 2007/134578

(56) Entgegenhaltungen:
- WO-A-01/60814
- WO-A-2006/052936

## Beschreibung

Die Erfindung betrifft neue Indol-Pyrrol-Derivate, pharmazeutische Zusammensetzungen enthaltend solche Verbindungen, Verwendungen solcher Verbindungen, sowie Verfahren zur Herstellung solcher Verbindungen.

Aus den Literaturstellen Sun, L. et al, J. Med. Chem. 46:1116-1119 (2003), Manley, J.M. et al, J. Org. Chem. 68:6447-6450 (2003) und Mendel, D.B. et al, Clin Cancer Res. 9(1):327-337 (2003) sind verschiedene Indol-Pyrrol-Derivate bekannt. Diese eigenen sich für die Behandlung verschiedener Krebsarten, Mastocystosis, Allergieassoziierte chronische Rhinitis, Diabetis, Arthritis, Angiogenese sowie verschiedene immunologische und cardiovaskuläre Erkrankungen.

Grundsätzlich besteht ein starker Bedarf nach neuen und verbesserten Wirksubstanzen, die in der Lage sind, die Proliferation von Krebszellen und somit das Wachstum neoplastischer Tumore zu hemmen sowie überschießende Abwehrreaktionen des Körpers, wie z.B. septischer Schock, Allergien, Autoimmunerkrankungen, Transplantatabstoßungen sowie akute und chronische Entzündungsreaktionen zu inhibieren, und zwar bei gleichzeitig lediglich geringfügiger bis keiner Zytotoxizität gegenüber intakten Zellen. Zusätzlich soll das Wachstum von unizellulären Organismen gehemmt werden.

Hierzu lehrt die Erfindung eine Verbindung gemäß Formel I: 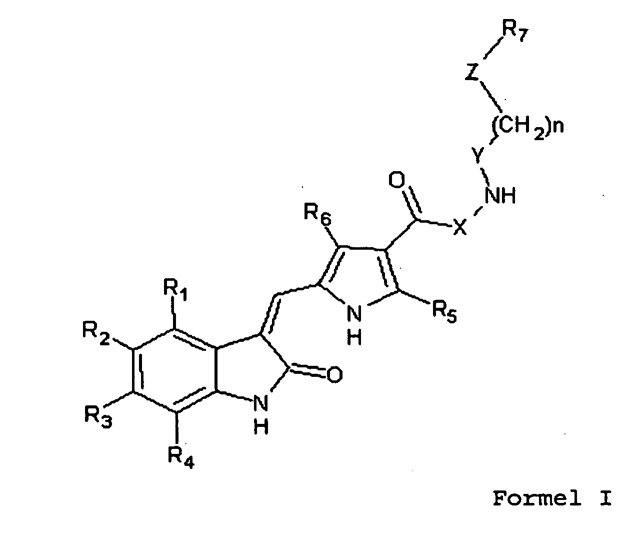 wobei R1 bis R4 gleich oder verschieden und -H, -F, - Cl, -Br, -I, oder (C₁-C₈)-Oxyalkyl sein können,
wobei R5 und R6 gleich oder verschieden und -H oder Cl-C4-Alkyl sein können,
wobei X für eine Bindung steht,
wobei Y und Z für eine chemische Bindung stehen können und wobei entweder Y oder Z für -O- steht,
wobei n = 0 bis 8 sein kann, und
wobei R7 eine -NR8R9 Gruppe,
in welcher R8 und R9 gleich oder verschieden und -H, eine (C₁-C₁₀)-Alkylgruppe oder (C₁-C₆)-Oxyalkyl sein können, eine (C₁-C₁₀)-Alkylgruppe oder eine gegebenenfalls teilweise oder vollständig halogenierte, insbesondere fluorierte, (C₁-C₁₀)-Alkylgruppe, (C₃-C₇)-Cycloalkylgruppe, (C₂-C₁₀)-Alkenylgruppe, (C₂-C₁₀)-Alkinylgruppe, (C₁-C₈)-Alkyl (C₃-C₇)cycloalkylgruppe, (C₂-C₈)-Alkenyl(C₃-C₇)cycloalkylgruppe, Heterocyclylgruppe, (C₁-C₈)-Alkylheterocyclylgruppe, (C₂-C₈)-Alkenylheterocyclylgruppe, Arylgruppe, (C₁-C₈)-Alkylarylgruppe, (C₂-C₈)-Alkenylarylgruppe, oder (C₂-C₈)-Alkinylarylgruppe, oder eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)-Alkylheteroarylgruppe, oder eine (C₂-C₈)-Alkenylheteroarylgruppe, eine (C₂-C₈)-Alkinylheteroarylgruppe ist, wobei diese Gruppen über eine beliebige Position mit R2 verknüpft sein können und gegebenenfalls an einer oder mehreren Stellen hydriert sein können,
und Stereoisomere sowie physiologisch verträgliche Salze solcher Verbindungen.

Vorzugsweise sind R1, R3 und R4 -H und R2 -F, -Cl, -Br, -I, oder (C₁-C₈)-Oxyalkyl. R5 und R6 können (C₁-C₃)-Alkyl, insbesondere Methyl, sein.

R7 ist vorzugsweise eine -NR8R9 Gruppe, in welcher R8 und R9 gleich oder verschieden und -H, eine (C₁-C₁₀)-Alkylgruppe oder (C₁-C₆)-Oxyalkyl sein können, eine (C₁-C₁₀)-Alkylgruppe oder eine gegebenenfalls teilweise oder vollständig halogenierte, insbesondere fluorierte, (C₁-C₁₀)-Alkylgruppe, (C₃-C₇)-Cycloalkylgruppe, (C₂-C₁₀)-Alkenylgruppe, (C₂-C₁₀)-Alkinylgruppe, (C₁-C₈)-Alkyl(C₃-C₇)cycloalkylgruppe, (C₂-C₈)-Alkenyl(C₃-C₇)cycloalkylgruppe, Heterocyclylgruppe, (C₁-C₈)-Alkylheterocyclylgruppe, (C₂-C₈)-Alkenylheterocyclylgruppe, Arylgruppe, (C₁-C₈)-Alkylarylgruppe, (C₂-C₈)-Alkenylarylgruppe, oder (C₂-C₈)-Alkinylarylgruppe, oder eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)-Alkylheteroarylgruppe, oder eine (C₂-C₈)-Alkenylheteroarylgruppe, eine (C₂-C₈)-Alkinylheteroarylgruppe, wobei diese Gruppen über eine beliebige Position mit R2 verknüpft sein können und gegebenenfalls an einer oder mehreren Stellen hydriert sein können. Im Einzelnen ist R7 bevorzugt eine -NR8R9 Gruppe, in welcher R8 und R9 gleich oder verschieden und -H, Methyl, Ethyl, Methoxy oder Ethoxy sind, oder pyrrolidin-1-yl, morpholin-4-yl, pyridin-4-yl, 1-methyl-4-piperidyl, oder triazol-1-yl, optional mit (C₁-C₆)-Alkyl, -F, -Cl, -Br, -I, oder (C₁-C₆)-Oxyalkyl einfach oder mehrfach substituiert.

Die Alkylgruppen für die beschriebenen Reste können geradkettig oder verzweigt sein und beispielsweise für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe, sowie die Hexyl-, Heptyl-, Nonyl-, Decylgruppe und ihre beliebig verzweigten Derivate stehen. Eine Methyl- oder Ethylgruppe ist bevorzugt. Die genannten Alkylgruppen können gegebenenfalls substituiert sein durch 1-5 Halogenatome. Für eine teilweise oder vollständig halogenierte, insbesondere fluorierte C₁-C₃-Alkylgruppe, kommen zum Beispiel die folgenden teilweise oder vollständig fluorierten folgenden Gruppen in Betracht: Fluormethyl, Difluormethyl, Trifluormethyl, Fluorethyl, 1,1-Difluorethyl, 1,2-Difluorethyl, 1,1,1-Trifluorethyl, Tetrafluorethyl, Pentafluorethyl. Von diesen bevorzugt sind die Trifluormethyl- oder die Pentafluorethylgruppe, wobei die vollständig fluorierte Gruppe auch Perfluoralkylgruppe genannt wird.

Die Alkoxygruppen (= Oxyalkyl) können geradkettig oder verzweigt sein und beispielsweise für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. C₁-C₅-Alkoxygruppen sind bevorzugt. Eine Methoxy- oder Ethoxygruppe ist besonders bevorzugt.

Die Cycloalkylgruppe bedeutet eine gegebenenfalls durch ein oder mehrere Halogenatome, (C₁-C₅)-Alkylgruppen, (C₁-C₅)-Alkoxygruppen, NR¹⁰R¹¹-Gruppen, COOR¹²-Gruppen, CHO, Cyano, substituierte gesättigte zyklische Gruppe mit 3 bis 7 Ringkohlenstoffatomen wie beispielsweise Cyclopropyl, Methylcyclopropyl, Cyclobutyl, Methylcyclobutyl, Cylopentyl, Methylcyclopentyl, Cyclohexyl, Methylcyclohexyl, Cycloheptyl, Methylcycloheptyl.

Unter einer (C₁-C₈)Alkyl(C₃-C₇)cycloalkylgruppe ist ein Cycloalkyl-Gruppe zu verstehen, die über eine geradkettige oder verzweigte (C₁-C₈)-Alkyleinheit mit dem Ringsystem verknüpft ist.

Unter einer (C₂-C₈)Alkenyl(C₃-C₇)cycloalkylgruppe ist ein Cycloalkyl-Gruppe zu verstehen, die über eine geradkettige oder verzweigte (C₂-C₈)-Alkenyleinheit mit dem Ringsystem verknüpft ist.

Die Heterocyclylgruppe ist nicht aromatisch und kann beispielsweise Pyrrolidin, Imidazolidin, Pyrazolidin, Piperidin sein. Auch Perhydrochinolin und Perhydroisochinolin gehören zu den mit umfaßten Heterocyclylgruppen.

Arylgruppen in Sinne der Erfindung sind aromatische oder teilaromatische carbocyclische Gruppen mit 6 bis 14 Kohlenstoffatomen, die einen Ring, wie z.B. Phenyl oder Phenylen oder mehrere kondensierte Ringe wie z.B. Napthyl oder Anthranyl aufweisen. Beispielhaft seien Phenyl, Naphthyl, Tetralinyl, Anthranyl, Indanyl, und Indenyl genannt.

Die Arylgruppen können an jeder geeigneten Stelle, die zu einem stabilen Stereoisomeren führt, substituiert sein durch einen oder mehrere Reste aus der Gruppe Hydroxy oder Halogen.

Eine (C₁-C₈)Alkylarylgruppe ist eine Arylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylarylgruppe ist eine Arylgruppe, wie sie oben bereits beschrieben ist, die über eine geradkettige oder verzweigte (C₂-C₈)-Alkenyleinheit mit dem Ringsystem verknüpft ist.

Eine (C₂-C₈)Alkinylarylgruppe ist eine Arylgruppe, wie sie oben bereits beschrieben ist, die über eine geradkettige oder verzweigte (C₂-C₈)-Alkinyleinheit mit dem Ringsystem verknüpft ist.

Monozyklische Heteroarylgruppen können beispielsweise Pyridin, Pyrazin, Pyrimidin, Pyridazin, Triazin, Azaindolizin, 2H- und 4H-Pyran, 2H- und 4H-Thiopyran, Furan, Thiophen, 1H- und 4H-Pyrazol, 1H- und 2H-Pyrrol, Oxazol, Thiazol, Furazan, 1H- und 4H-Imidazol , Isoxazol, Isothiazol, Oxadiazol, Triazol, Tetrazol, Thiadiazol sein.

Bizyklische Heteroarylgruppen können beispielsweise Phthalidyl-, Thiophthalidyl-, Indolyl-, Isoindolyl-, Dihydroindolyl-, Dihydroisoindolyl-, Indazolyl-, Benzothiazolyl-, Indolonyl-, Dihydroindolonyl-, Isoindolonyl-, Dihydroisoindolonyl-, Benzofuranyl-, Benzimidazolyl-, Dihydroisochinolinyl-, Dihydrochinolinyl-, Benzoxazinonyl-, Phthalazinonyl-, Dihydrophthalazinonyl-Chinolinyl-, Isochinolinyl-, Chinolonyl-, Isochinolonl-, Chinazolinyl-, Chinoxalinyl-, Cinnolinyl-, Phthalazinyl-, Dihydrophthalazinyl-, 1,7- oder 1,8-Naphthyridinyl-. Cumarinyl-, Isocumarinyl-, Indolizinyl-, Isobenzofuranyl-, Azaindolyl-, Azaisoindolyl-, Furanopyridyl-, Furanopyrimidinyl-, Furanopyrazinyl-, Furanopyidazinyl-, Dihydrobenzofuranyl-, Dihydrofuranopyridyl-, Dihydrofuranopyrimidinyl-, Dihydrofuranopyrazinyl-, Dihydrofuranopyridazinyl-, Dihydrobenzofuranyl-, Chromenyl-, Isochromenyl-, Chromenonyl- oder die Isochromenonylgruppe sein.

Eine (C₁-C₈)Alkylheteroarylgruppe ist eine Heteroarylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylheteroarylgruppe ist eine Heteroarylgruppe, wie sie oben bereits beschrieben ist, die über eine geradkettige oder verzweigte (C₂-C₈)-Alkenyleinheit mit dem Ringsystem verknüpft ist.

Eine (C₂-C₈)Alkinylheteroarylgruppe ist eine Heteroarylgruppe, wie sie oben bereits beschrieben ist, die über eine geradkettige oder verzweigte (C₂-C₈)-Alkinyleinheit mit dem Ringsystem verknüpft ist.

Eine (C₁-C₈)Alkylheterocyclylgruppe ist eine Heterocyclylgruppe, wie sie oben bereits beschrieben ist, die über eine geradkettige oder verzweigte (C₁-C₈)-Alkyleinheit mit dem Ringsystem verknüpft ist.

Eine (C₂-C₈)Alkenylheterocyclylgruppe ist eine Heterocyclylgruppe, wie sie oben bereits beschrieben ist, die über eine geradkettige oder verzweigte (C₂-C₈)-Alkenyleinheit mit dem Ringsystem verknüpft ist.

Beispiele für erfindungsgemäße Verbindungen sind:
5-[5-Fluoro-2-oxo-1,2-dihydro-indol-(3Z)-ylidenemethyl]-2,4-dimethyl-1H-pyrrole-3-carboxylic acid (2-diethylamino-ethoxy)-amide,
5-[5-Bromo-2-oxo-1,2-dihydro-indol-(3Z)-ylidenemethyl]-2,4-dimethyl-1H-pyrrole-3-carboxylic acid (2-diethylamino-ethoxy)-amide,
5-[5-Chloro-2-oxo-1,2-dihydro-indol-(3Z)-ylidenemethyl]-2,4-dimethyl-1H-pyrrole-3-carboxylic acid (2-diethylamino-ethoxy)-amide,
5-[6-Fluoro-2-oxo-1,2-dihydro-indol-(3Z)-ylidenemethyl]-2,4-dimethyl-1H-pyrrole-3-carboxylic acid (2-diethylamino-ethoxy)-amide,
5-[6-Chloro-2-oxo-1,2-dihydro-indol-(3Z)-ylidenemethyl]-2,4-dimethyl-1H-pyrrole-3-carboxylic acid (2-diethylamino-ethoxy)-amide,
5-[6-Bromo-2-oxo-1,2-dihydro-indol-(3Z)-ylidenemethyl]-2,4-dimethyl-1H-pyrrole-3-carboxylic acid (2-diethylamino-ethoxy)-amide,
5-[5-Methyl-2-oxo-1,2-dihydro-indol-(3Z)-ylidenemethyl]-2,4-dimethyl-1H-pyrrole-3-carboxylic acid (2-diethylamino-ethoxy)-amide,
5-[5-Methoxy-2-oxo-1,2-dihydro-indol-(3Z)-ylidenemethyl]-2,4-dimethyl-1H-pyrrole-3-carboxylic acid (2-diethylamino-ethoxy)-amide, oder
5-[6-Methoxy-2-oxo-1,2-dihydro-indol-(3Z)-ylidenemethyl]-2,4-dimethyl-1H-pyrrole-3-carboxylic acid (2-diethylamino-ethoxy)-amide,
oder Derivate der vorstehenden Verbindungen, wobei die 2-diethylamino-ethoxy-Gruppe ersetzt ist durch eine der Gruppen ausgewählt aus "2-(triazol-1-yl)ethoxy, 2-triazol-1-yl)methoxy, (pyridin-4-yl)methoxy, 2-(pyridiny-4-yl)ethoxy, 2-(morpholin-4-yl)ethoxy, (morpholin-4-yl)methoxy, (1-methyl-4-piperidyl)methoxy, 2-(1-methyl-4-piperidyl)ethoxy, 2-(pyrrolidin-1-yl)ethoxy, (pyrrolidin-1-yl)methoxy, 2-(dimethylamino)ethoxy, (dimethylamino)methoxy und (dimethylamino)methoxy".

In allen vorstehenden konkreten Verbindungen sind X und Z eine Bindung und Y ist -O-. Des weiteren kann in allen vorstehenden konkreten Verbindungen -Y- eine Bindung sein, wenn X -O- ist.

Im Rahmen von pharmazeutischen Zusammensetzungen können erfindungsgemäße Verbindungen mit anderen, per se bekannten Wirkstoffen kombiniert werden. Hierfür kommen beispielsweise in Frage: Aldesleukin, Amifostine, Atrasentan, Bevacizumab, Bexaroten, Bortezomib, Capecitabine, Carboplatin, Chlorambucil, Cisplatin, Cladribine, Cyclophosphamid, Cytamid, Dacarbazin, Docetaxel, Droloxifene, Edrecolomab, Epothilone, Erlotinib, Etoposide, Exemestane, Flavopiridol, Fludarabine, Fuorouracil, Formestane, Fulvestrant, Gefitinib, Gemcitabine, Idarubicin, Irinotecan, Ixabepilone, Lonafarnib, Miltefosine, Mitomycin, Neovastat, Oxaliplatin, Pemetrexed, Porfimer, Rituximab, Tegafur, Temozolomide, Tipifarnib, Topotecan, Trimetrexate, Vorozole, Vinblastine, und Mischungen von zwei oder mehreren solcher Wirkstoffe. Dabei kann die erfindungsgemäße Verbindung in Rahmen einer einzigen galenischen Herrichtung mit der Wirksubstanz gemischt sein. Es ist aber auch möglich, dass die pharmazeutische Zusammensetzung aus zwei (oder mehr) verschiedenen galenischen Herrichtungen besteht, wobei in einer ersten Herrichtung die erfindungsgemäße Verbindung und in einer zweiten Herrichtung der Wirkstoff enthalten sind. Dabei kann im Rahmen der ersten Herrichtung auch ein von dem Wirkstoff der zweiten Herrichtung verschiedener Wirkstoff eingerichtet sein.

Die Erfindung betrifft des weiteren ein Verfahren zur Herstellung erfindungsgemäßer Verbindungen wobei eine Substanz der Formel II 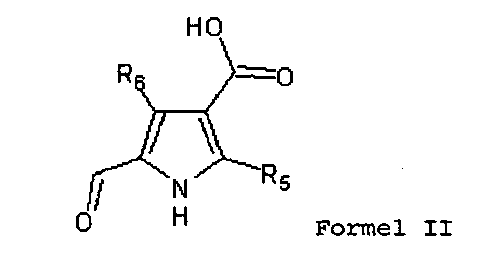 mit einer Substanz der Formel III 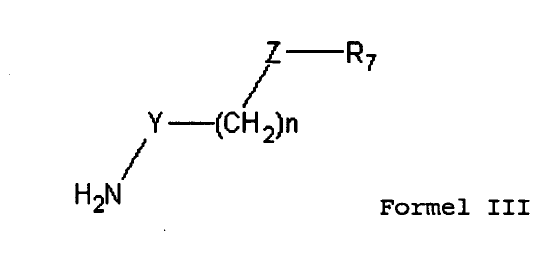 zu einer Substanz der Formel IV 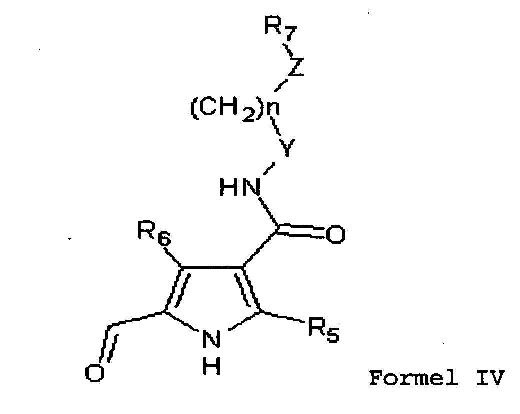 umgesetzt wird, wobei dann die Substanz der Formel IV mit einer Substanz der Formel V 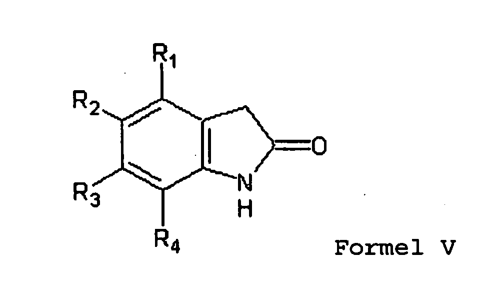 zu einer Verbindung der Formel VI 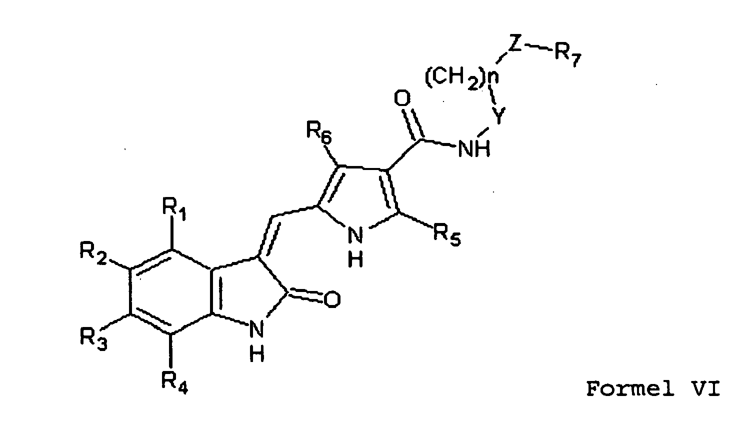 umgesetzt wird, wobei die Reste R1 bis R7 sowie die Gruppen Y und Z die vorstehenden Bedeutungen haben und wobei vorzugsweise Y -O- ist.

Substanzen der Formel II lassen sich beispielsweise durch Umsetzung von Verbindungen der Formeln VII und VIII oder der Formeln IX und X miteinander und anschließender Umsetzung zur Substanz der Formel II synthetisieren, wobei zumindest die jeweilige reaktive Gruppe Y oder Z -O- ist.

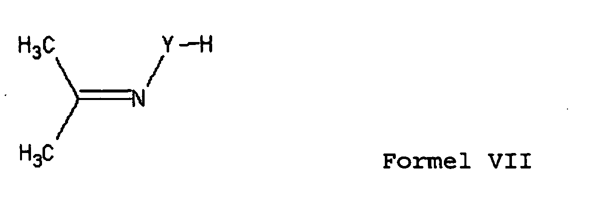

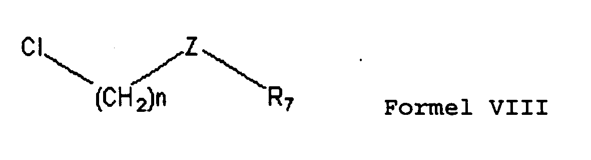

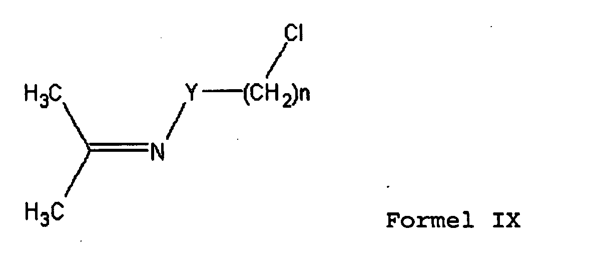

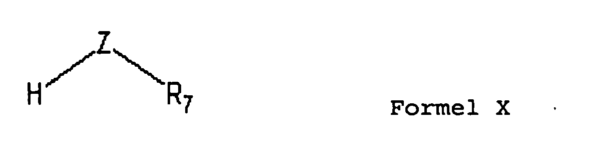

Beispielhafte geeignete Reaktionsbedingungen für die vorstehenden Umsetzungen sind den Ausführungsbeispielen entnehmbar.

Erfindungsgemäße Verbindungen sind beispielsweise gemäß dem folgenden Syntheseschema herstellbar:

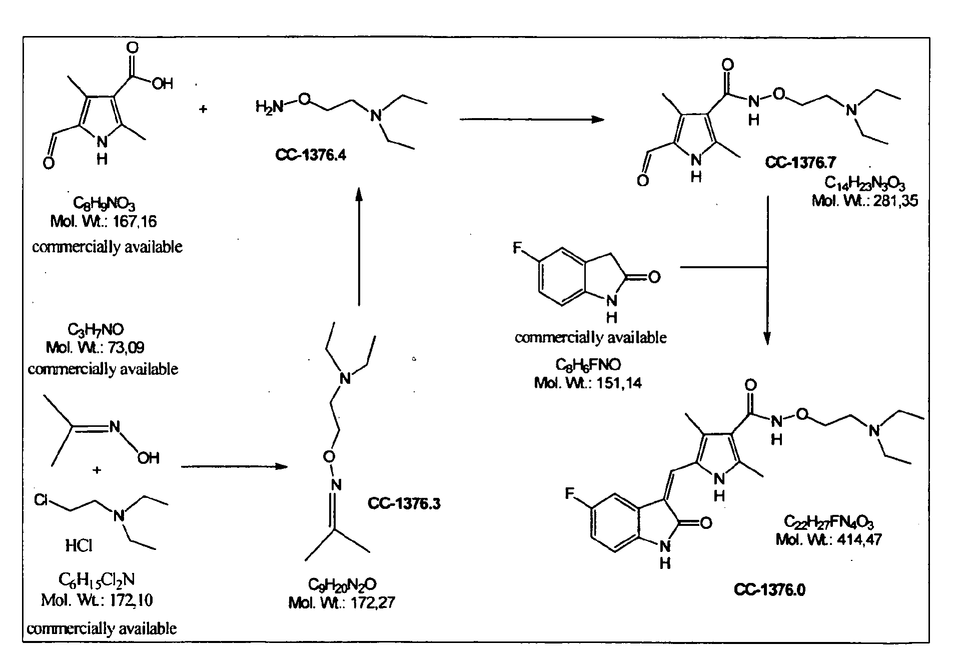

Andere Verbindungen der Erfindung können analog hergestellt werden durch Einsatz von Edukten, bei welchen die Reste R1 bis R7 definitionsgemäß modifiziert sind.

Die Erfindung lehrt des weiteren eine pharmazeutische Zusammensetzung enthaltend eine erfindungsgemäße Verbindung. Optional können ein oder mehrere physiologisch verträgliche Hilfstoffe und/oder Trägerstoffe mit der Verbindung gemischt und die Mischung galenisch zur lokalen oder systemischen Gabe, insbesondere oral, parenteral, zur Infusion bzw. Infundierung in ein Zielorgan, zur Injektion (z.B. i.v., i.m., intrakapsulär oder intralumbal), zur Applikation in Zahntaschen (Raum zwischen Zahnwurzel und Zahnfleisch) und/oder zur Inhalation hergerichtet ist. Die Auswahl der Zusatz- und/oder Hilfsstoffe wird von der gewählten Darreichungsform abhängen. Die galenische Herrichtung der erfindungsgemäßen pharmazeutischen Zusammensetzung kann dabei in fachüblicher Weise erfolgen. Als Gegenionen für ionische Verbindungen kommen beispielsweise Ca⁺⁺, CaCl⁺, Na⁺, K⁺, Li⁺ oder Cyclohexylammonium, bzw. Cl⁻, Br⁻, Acetat, Trifluoracetat, Propionat, Laktat, Oxalat, Malat, Maleat, Malonat, Maleinat, Citrat, Benzoat, Salicylat usw. in Frage. Geeignete feste oder flüssige galenische Zubereitungsformen sind beispielsweise Granulate, Pulver, Dragees, Tabletten, (Mikro-) Kapseln, Suppositorien, Sirupe, Säfte, Suspensionen, Emulsionen, Tropfen oder Lösungen zur Injektion (i.v., i.p., i.m., s.c.) oder Vernebelung (Aerosole), Zubereitungsformen zur Trockenpulverinhalation, transdermale Systeme, sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Auch ist es möglich, den Wirkstoff in vorzugsweise biologisch abbaubaren Nanokapseln zu verkapseln, beispielsweise zur Herstellung einer Zubereitung zur Inhalation. Als Hilfsstoffe seien beispielsweise Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt. Eine erfindungsgemäße pharmazeutische Zusammensetzung ist dadurch herstellbar, dass mindestens ein erfindungsgemäß verwendete Substanzkombination in definierter Dosis mit einem pharmazeutisch geeigneten und physiologisch verträglichen Träger und ggf. weiteren geeigneten Wirk-, Zusatz- oder Hilfsstoffen mit definierter Dosis gemischt und zu der gewünschten Darreichungsform hergerichtet ist.

Als Verdünnungsmittel kommen Polyglykole, Ethanol, Wasser und Pufferlösungen in Frage. Geeignete Puffersubstanzen sind beispielsweise N,N'-Dibenzylethylendiamin, Diethanolamin, Ethylendiamin, N-Methylglucamin, N-Benzylphenethylamin, Diethylamin, Phosphat, Natriumbicarbonat, oder Natriumcarbonat. Es kann aber auch ohne Verdünnungsmittel gearbeitet werden.

Physiologisch verträgliche Salze sind Salze mit anorganischen oder organischen Säuren, wie Salzsäure, Schwefelsäure, Essigsäure, Weinsäure, Milchsäure, Citronensäure, Maleinsäure, p-Toluolsulfonsäure, und insbesondere Äpfelsäure, oder mit anorganischen oder organischen Basen, wie NaOH, KOH, Mg(OH)₂, Diethanolamin, Ethylendiamin, oder mit Aminosäuren, wie Arginin, Lysin, Glutaminsäure usw. oder mit anorganischen Salzen, wie CaCl₂, NaCl oder deren freie Ionen, wie Ca²⁺, Na⁺, Cl⁻, SO₄²⁻ oder Kombinationen hieraus. Sie werden nach Standardmethoden hergestellt. Ergänzend wird auf geeignete Gegenionen, wie im Zusammenhang mit der galenischen Herrichtung genannt, verwiesen.

Die Erfindung beruht auf der Erkenntnis, daß durch die Einführung zumindest eines -O- für eine der Gruppen X, Y, oder Z eine verbesserte Wirksamkeit erhalten wird, da Verbindungen mit -N-O- Gruppen einerseits kompetitiv natürlichen Liganden binden und andererseits nicht verstoffwechselt werden können. Die inhibitorische Wirkung wird also erheblich erhöht.

Die Erfindung lehrt weiterhin die Verwendung einer erfindungsgemäßen Verbindung zur Herstellung einer pharmazeutischen Zusammensetzung zur Behandlung einer oder mehrerer Erkrankungen aus der Gruppe bestehend aus "Krebs, wie Lungenkrebs, Leukämie, Eierstockkrebs, Sarkome, Meningiom, Darmkrebs, Lyphknotenkrebs, Hirntumore, Brustkrebs, Pankreaskrebs, Prostatakrebs, Hautkrebs, chronische Entzündungen, Asthma, Allergie, Rhinitis, Uveitis, Urticaria, Arthritis, Osteaoarthritis, chronische Polyarthritis, rheumatoide Arthritis, Inflammatory bowl disease, degenerative Gelenkserkrankungen, Erkrankungen des rheumatischen Formenkreises mit Knorpelabbau, Sepsis, Autoimmunerkrankungen, Typ I Diabetes, Hashimoto-Thyreoiditis, Autoimmunthrombozytopenie, Multiple Sklerose, Myasthenia gravis, chronisch entzündliche Darmerkrankungen, Morbus Crohn, Uveitis, Psoriasis, atypische Dermatitits, Kollagenosen, Goodpasture-Syndrom, Erkrankungen mit gestörter Leukozyten-Adhäsion, Cachexie, Erkrankungen durch erhöhte TNFalpha Konzentration, Diabetes, Adipositas, bakterielle Infektionen, insbesondere mit resistenten Bakterien, Herzinsuffizienz und der Chronic Cardiac Failure (CCF)". Der Begriff der Behandlung umfaßt auch die Prophylaxe.

Im Rahmen der Erfindung sind diverse weitere Ausführungsformen möglich. So kann eine erfindungsgemäße pharmazeutische Zusammensetzung mehrere verschiedene, unter die vorstehende Formel I fallende Verbindungen enthalten. Weiterhin kann eine erfindungsgemäße pharmazeutische Zusammensetzung zusätzlich einen von der Verbindung der Formel I verschiedenen Wirkstoff enthalten. Dann handelt es sich um ein Kombinationspräparat. Dabei können die verschiedenen eingesetzten Wirkstoffe in einer einzigen Darreichungsform präpariert sein, i.e. die Wirkstoffe sind in der Darreichungsform gemischt. Es ist aber auch möglich, die verschiedenen Wirkstoffe in räumlich getrennten Darreichungsformen gleicher oder verschiedener Art herzurichten.

Die Erfindung betrifft auch ein Verfahren zur Herstellung einer pharmazeutischen Zusammensetzung, wobei mindestens eine erfindungsgemäße Verbindung mit einem pharmazeutisch geeigneten und physiologisch verträglichen Trägerstoff und gegebenenfalls weiteren geeigneten Wirk-, Zusatz- oder Hilfsstoffen gemischt und in eine geeignete Darreichungsform gebracht wird.

Vorzugsweise wird die pharmazeutische Zusammensetzung in Dosierungseinheiten hergestellt und verabreicht, wobei jede Einheit als aktiven Bestandteil eine definierte Dosis der erfindungsgemäßen Verbindung gemäß Formel I enthält. Bei festen Dosierungseinheiten wie Tabletten, Kapseln, Dragees oder Suppositorien kann diese Dosis 0,1 bis 1000 mg, bevorzugt 1 bis 300 mg, und bei Injektionslösungen in Ampullenform 0,01 bis 1000 mg, vorzugsweise 1 bis 100 mg, betragen.

Für die Behandlung eines Erwachsenen, 50 bis 100 kg schweren, beispielsweise 70 kg schweren, Patienten sind beispielsweise Tagesdosen von 0,1 bis 1000 mg Wirkstoff, vorzugsweise 1 bis 500 mg, indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Möglich ist auch eine Kombination eines oder mehrerer der erfindungsgemäßen Wirkstoffe mit Aminooxyacetat (AOA, NH2-O-CH2-COOH oder dessen Salze oder Ester, beispielsweise C1 -C10 Alkyl- oder Hydroxyalkylester). AOA ist insbesondere auf kleine Tumore (< 0,1 bis 1 cm³) wirksam bzw. verhindert deren Bildung, insbesondere die Metastasenbildung, während erfindungsgemäße Verbindungen insbesondere gegen die großen Tumore wirksam ist. Grund hierfür sind die unterschiedlichen Stoffwechsel in kleinen bzw. großen Tumoren. Die vorstehenden Ausführungen zu Kombinationen gelten analog.

Im Folgenden werden Synthesebeispiele für erfindungsgemäße Verbindungen angegeben.

### 1.1 Synthese von Acetone β-Diethylaminoethyl Oxime (CC-1376.3)

### Reaktion:

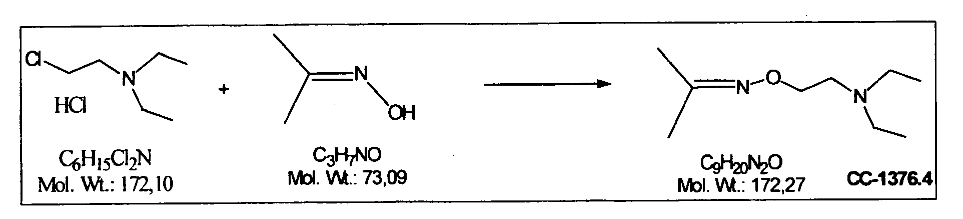

Ansatz CC-1376.3-1:

Natrium (EH-122.5-5, 18,4 g, 0,8 mol) wird in 600 ml trockenem Ethanol unter einer Argonatmospäre gelöst. Nach Zugabe von Acetoxim (29,2 g, 0,4 mol) und Diethylaminoethylchlorid Hydrochlorid (EH-873.3-2, 68,8 g, 0,4 mol) wird auf Rückfluss erhitzt. Nach 2h Rühren wird das Heizbad entfernt und über Nacht bei RT gerührt. Nach Filtration wird mit 2 N wässrigen HCl angesäuert (pH 5) und im Vakuum eingeengt. Der Rückstand wird mit 10% iger NaOH aufgenommen, 2 x mit je 250 ml Diethylether extrahiert und die organischen Phasen im Vakuum eingeengt. Nach Vakuumdestillation (56 - 68 °C, ca. 6 mbar) erhält man CC-1376.3-1 (43,94 g, 64%).

### 1.2 Synthese von β-Diethylaminoethoxyamine (CC-1376.4) Reaktion:

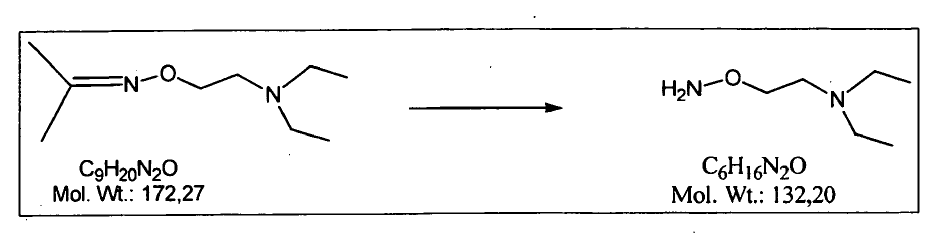

Ansatz CC-1376.4-1:

Acetone β-Diethylaminoethyl Oxime (CC-1376.3-1, 43,94 g, 255 mmol) wird in 3N wässrigen HCl (450 ml) versetzt und auf Rückfluss gebracht. Nach 3h Rühren bei dieser Temperatur wird abgekühlt und im Vakuum eingeengt. Der Rückstand wird mit einer 10 % igen und 50% igen Natriumhydroxid-Lösung alkalisch gestellt. Die wässrige Phase wird 2 x mit je 250 ml Diethylether extrahiert, über Natriumsulfat getrocknet und im Vakuum eingeengt. Nach Vakuumdestillation (62 - 66 °C, ca. 6 mbar) erhält man CC-1376.4-1 (27,2g, 81%) leicht verunreinigt, das ohne weitere Reinigung in der nächsten Stufe eingesetzt wird.

### 1.3 Synthese von 5-Formyl-2,4-dimethyl-1H-pyrrole-3-carboxylic acid (2-diethylaminoethoxy)-amide (CC-1376.7)

### Reaktion:

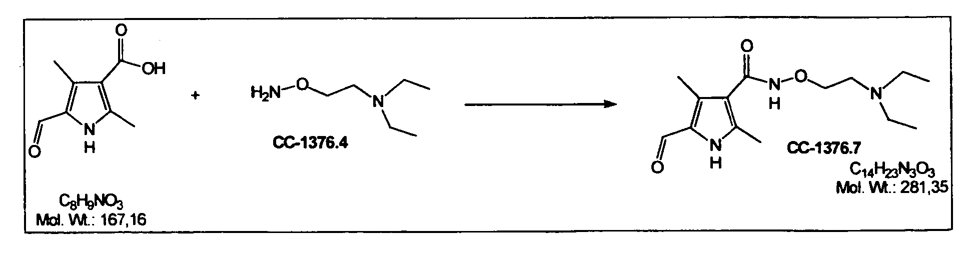

Ansatz CC-1376.7-1:

Hydroxybenzotriazol (EH-1100.2-2, 300 mg, 2,22 mmol), EDC1 (430 mg, 2,24 mmol) und 3,5-Dimethyl-2-formylpyrrole-4-carbonsäure (EH-1195.10-1, 250 mg, 1,5 mmol) werden unter Argonatmosphäre bei RT in 10 ml trockenem DMF vorgelegt und mit Triethylamin (EH-18.3-13, 0,42 ml, 3 mmol) versetzt. Anschließend tropft man zügig CC-1376.4-1 (400 mg, 3 mmol) zu und rührt bei RT über Nacht. Die Reaktionslösung wird mit 5 ml Wasser, 3 ml ges. NaCl und 5 ml ges. NaHCO₃ Lösung. Verdünnt, 2 x mit je 10 ml DCM/MeOH 9:1 extrahiert, über Natriumsulfat getrocknet und im Vakuum eingeengt. Der Rückstand wird mit Toluol koevaporiert, mit 10 ml Hexan/Diethylether 3:1 versetzt und die überstehende Lösung abdekantiert. Der Rückstand wird erneut mit Toluol koevaporiert und nach Reinigung über Flash-Kieselgel (DCM/MeOH 1:1) und Trocknung im Hochvakuum erhält man CC-1376.7-1 als braunen Feststoff (114 mg, 28%).

### 1.4 Synthese von 5-[5-Fluoro-2-oxo-1,2-dihydro-indol-(3Z)-ylidenemethyl]-2,4-dimethyl-1H-pyrrole-3-carboxylic acid (2-diethylamino-ethoxy)-amide (CC-1376.0)

### Reaktion:

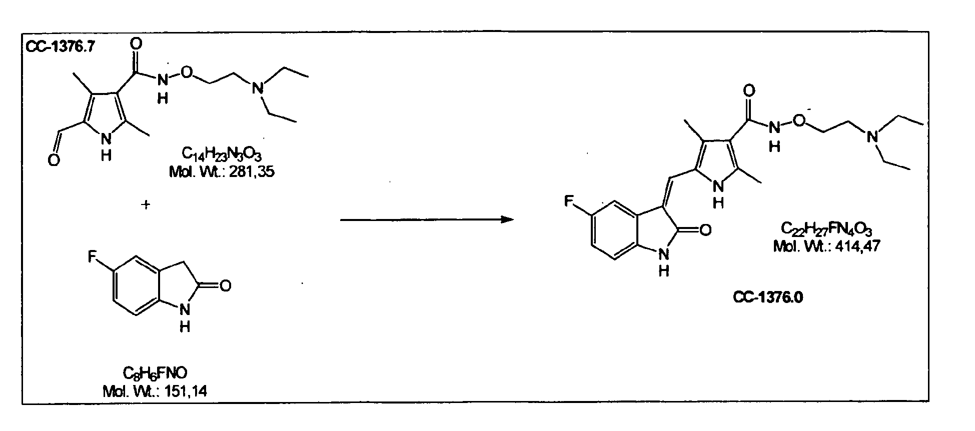

Ansatz CC-1376.0-3:

5-Fluoroxindol (0,33 g, 2,18 mmol) wird in 12 ml EtOH gelöst. Anschließend gibt man 5-Formyl-2,4-dimethyl-1H-pyrrole-3-carbonsäure-(2-diethylaminoethoxy)-amide (CC-1376.7-2, 620 mg, 2,20 mmol) und Pyrrolidin (18 µl, 0,22 mmol) zu. Nach Zugabe erwärmt man 3h auf 78 °C, wobei sich ein orangener Feststoff abscheidet. Die Reaktionsmischung wird abgekühlt und der Niederschlag über eine Fritte abgesaugt, mit Ethanol nachgewaschen und erneut mit Ethanol bei 78 °C ausgerührt. Erneutes Filtrieren des Niederschlags und Trocknung im HV ergibt CC-1376.0-3 als gelben Feststoff (0,21g, 23%).

### 2: Strukturen und analytische Daten

### 2.1: 5-[5-Fluoro-2-oxo-1,2-dihydro-indol-(3Z)-ylidenemethyl]-2,4-dimethyl-1H-pyrrole-3-carboxylic acid (2-diethylamino-ethoxy)-amide

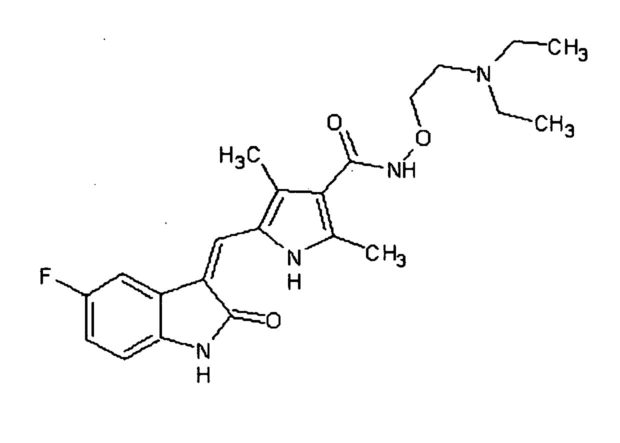

MW: 414,47 g/mol.
Schmelzpunkt: 216,0°C (Electrothermal IA 9200, Heizrate 1°C/min).

1H-NMR (gemessen mit Bruker Avance 400 (400 MHz, dmso-d₆, TMS als internen Standard): δ(ppm)= 0,96 (t, 6H, J=7,1Hz, Me), 2,43 (s, 3H, Me), 2,50 (m, 4H, NCH₂CH₃), 2,68 (t, 2H, J=6,1Hz, NCH₂CH₂), 3,92 (t, 2H, J=6,1Hz, NCH₂CH₂), 6,83-6,86 (m, 1H, H_{indol}), 6,91-6,96 (m, 1H, H_{indol}), 7,71 (s, 1H, C=CH), 7,75-7,79 (m, 1H,H_{indol}), 10,92 (bs, 1H, NH), 13,71 (bs, 1H, NH).

13C-NMR (gemessen mit Bruker Avance 400 (100,6 MHz, dmso-d₆, TMS als internen Standard): δ(ppm)=10,43 (s, 2C, NCH₂CH₃), 13,21 (s, 1C, Me), 46,75 (s, 2C, NCH₂CH₃), 50,43 (s, 1C, NCH₂CH₃), 73,69 (s, 1C, NCH₂CH₃), 105,97 (d, 1C, J=25,5Hz, CH-_{Indol}), 109,96 (d, 1C, J=8,6Hz, CH-_{Indol}), 112,45 (d, 1C, J=24,1Hz, CH-_{Indol}), 114,94 (d, 1C, J=3,8Hz), 117,51 (s, 1C), 124,72 (s, 1C), 125,85 (s, 1C, C=CH), 126,96 (d, 1C), 130,42 (s, 1C), 134,50 (d, 1C, J=1,3Hz), 136,52 (s, 1C), 156,97 (s, 1C), 159,30 (s, 1C), 169,47 (d, 1C, J=0,86Hz).

### 2.2: N-[2-(diethylamino)ethoxy]-5-[(Z)-(5-Fluoro-1,2-dihydro-2-oxo-3H-indol-3-ylidene)methyl]-2,4-dimethyl-1H-pyrrole-3-carboxamide, (2S)-hydroxybutanedionate (1:0,5)

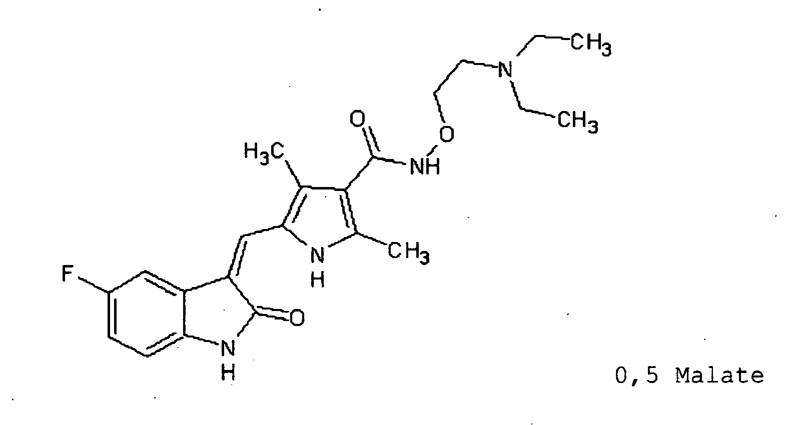

MW: 481,52 g/mol.
Schmelzpunkt: 203°C, Zersetzung (Electrothermal IA 9200, Heizrate 1°C/min).

1H-NMR (gemessen mit Bruker Avance 400 (400 MHz, dmso-d₆, TMS als internen Standard): δ(ppm)= 1,08 (t, 6H, J=7,0Hz, Me), 2,33 (dd, 0,5H, J=4,0Hz, J=15,6Hz, CH_{2malic}), 2,41 (s, 3H, Me), 2,44 (s, 3H, Me), 2,50 (m, 0,5H, CH_{2malic}) 2,84 (bs, 4H, NCH₂CH₃), 2, 97 (bs, 2H, NCH₂CH₂), 3, 90 (dd, 0, 5H, J=3,9Hz, J=10,0Hz CH_{malic}), 4,05 (m, 2H, J=6,1Hz, NCH₂CH₂), 6,85 (dd, 1H, J=4,6Hz, J=8,4Hz, H_{indol}), 6,89-6,98 (m, 1H, H_{indol}), 7,73 (s, 1H, C=CH), 7,78 (dd, 1H, J=2,4Hz, J=9,4Hz, H_{indol}), 10,94 (bs, 1H, NH), 13,76 (bs, 1H, NH).

### 2.3: N-[2-(diethylamino)ethoxy]-5-[(Z)-(5-Fluoro-1,2-dihydro-2-oxo-3H-indol-3-ylidene)methyl]-2,4-dimethyl-1H-pyrrole-3-carboxamide, (2S)-hydroxybutanedionate (1:1)

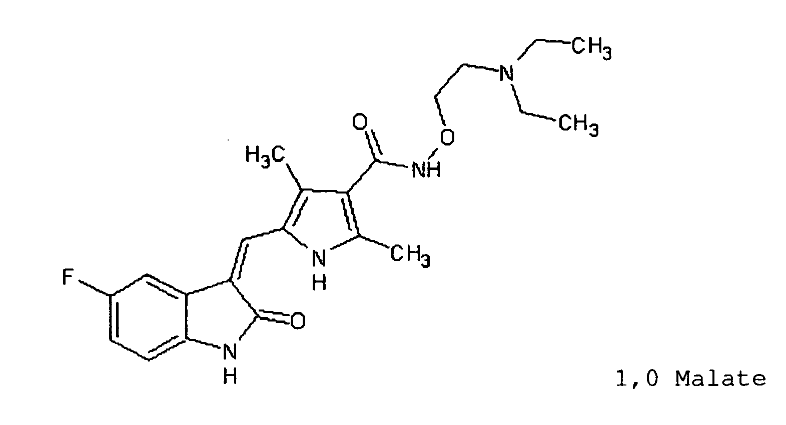

MW: 548,56 g/mol.

1H-NMR (gemessen mit Bruker Avance 400 (400 MHz, dmso-d₆, TMS als internen Standard) : δ(ppm)= 1, 12 (t, 6H, J=7,1Hz, Me), 2,34 (dd, 1H, J=4,0Hz, J=15,6Hz, CH_{2malic}), 2,42 (s, 3H, Me), 2,45 (s, 3H, Me), 2,50 (m, 1H, CH_{2malic}), 2,95 (bs, 4H, NCH₂CH₃), 3,08 (bs, 2H, NCH₂CH₂), 3,95 (dd, 1H, J=4,6Hz, J=9,1Hz CH_{malic}), 4,09 (m, 2H, NCH₂CH₂), 6,85 (dd, 1H, J=4,6Hz, J=8,5Hz, H_{indol}), 6,89-6,98 (m, 1H, H_{indol}), 7,73 (s, 1H, C=CH), 7,78 (dd, 1H, J=2,5Hz, J=9,4Hz, H_{indol}), 10,94 (bs, 1H, NH), 13,76 (bs, 1H, NH).

13C-NMR (gemessen mit Bruker Avance 400 (100,6 MHz, dmso-d₆, TMS als internen Standard): δ(ppm)=9,92 (s, 2C, NCH₂CH₃), 10,47 (s, 1C, Me), 13,29 (s, 1C, Me), 35,40 (s, 1C, NCH₂CH₂), 41,05 (s, 1C), 46,62 (s, 2C, NCH₂CH₃), 49,87 (s, 1C, NCH₂CH₂), 65,94 (s, 1C, CH_{malic}), 71,58 (d, 1C, NCH₂CH₂), 106,06 (d, 1C, J=26,0Hz, CH-_{Indol}), 110,03 (d, 1C, J=8,7Hz, CH-_{Indol}), 112,60 (d, 1C, J=23,9Hz, CH-_{Indol}), 115,30 (d, 1C, J=3,1Hz), 112,60 (d, 1C, J=23,9Hz, CH-_{Indol}), 115, 30 (d, 1C, J=3,1Hz), 116,71 (s, 1C), 124,67 (s, 1C, C=CH), 125,94 (s, 1C), 126,90 (d, 1C, J=9,5Hz), 130,34 (s, 1C), 134,57 (d, 1C, J=1,4Hz), 136,76 (s, 1C), 158,15 (d, 1C, J=234,3Hz), 163,75 (s, 1C), 169,47 (d, 1C, J=0,9Hz), 171,82 (s, 1C, CO), 175,97 (s, 1C, CO).

### 3: Biologische Daten

### 3.1: Zellkultur Ergebnisse

Die Substanzen der Beispiele 2.1 bis 2.3 wurden an verschiedenen Zelllinien in An- und Abwesenheit von Pyruvat im Nährmedium auf ihre proliferationshemmende Wirkung getestet. Die Substanzen wurden jeweils in DMSO gelöst. Anschließend wurde der Wirkstoff in acht Konzentrationsintervallen getestet. Der Berechnung der IC50-Werte lagen in jeder Konzentration acht Einzelwertbestimmungen zugrunde. Der IC50-Wert gibt die Wirkstoffkonzentration an, bei der im Vergleich zur Kontrollgruppe, die ausschließlich mit dem Lösungsmittel DMSO behandelt wurde, eine 50%ige Hemmung der Zellproliferation vorlag.

Die IC50-Werte für SO 272 lagen zwischen 1 und 11 µM. Die erhaltenen Werte sind in der Tabelle 1 angegeben.

**Tabelle 1**

| Zellinie (XTT) | Substanz | IC50 (XTT) | IC50 |
|---|---|---|---|
| | (aus Beispiel) | mit Pyrovat | ohne Pyrovat |
| MCF-7 | 2.1 | 11 | 8 |
| | 2.2 | 8 | 11 |
| | 2.3 | 9 | 10 |
| HT-29 | 2.1 | | 3,5 |
| | 2.2 | | 3 |
| | 2.3 | | 3 |
| BxPC-3 | 2.1 | 1,3 | 2 |
| | 2.2 | 2 | 2 |
| | 2.3 | 2 | 2 |
| MDA-MB-453 | 2.1 | 11 | 7 |
| | 2.2 | 8 | 7 |
| | 2.2 | 8 | 7 |
| NK1 | 2.1 | | 2 |
| | 2.2 | | 4 |
| | 2.3 | | 4 |
| Wi-38 | 2.1 | 11 | |
| KBV-600 | 2.1 | | 5 |

| | | | |
|---|---|---|---|
| MCF-7: humane Brustkrebs-Zelllinie; MDA-MB-453: humane Brustkrebs-Zelllinie; HT29: humane Colonkarzinom-Zelllinie; BxPC-3: humane Pankreastumor-Zelllinie; KBV600: Multidrugresistenter Abkömmling von HeLa-Zellen (KB-V1), kultiviert mit 600 ng/ml Vinblastin; WI -38: humane, fetale Fibroblasten-ähnliche Zelllinie der Lunge; NK: Novikoff-Ratten-Hepatoma-Zelllinie. | | | |

Die Substanzen hemmen gleich mehrere Rezeptoren von Thyrosinkinasen, die für Wachstum und Angiogenese der Tumoren wichtig sind. Der Multi-Thyrosinkinase-Hemmer ist z.B. auch für die Zweittherapie bei metastasierten Nierenzell-Karzinomen (RCC) oder mestastasierten gastrointestinalen Stromatumoren (GIST) bevorzugt geeignet. Ingesamt zeigen die Ergebnisse, dass mittels der erfindungsgemäßen Substanzen nicht nur spezifische Tumorarten behandelt werden können, sondern verschiedenste Tumorarten therapierbar sind.

### 3.2: Kolonie-Assays

Im Kolonie-Assay werden Tumorstammzellen von verschiedenen menschlichen Tumorentitäten in Softagar kultiviert und die Ausbildung von Tumorkolonien in An- und Abwesenheiten von der erfindungsgemäßen Substanz (Beispiel 2.1, ggf. als Malat der Beispiel 2.2 oder 2.3) ausgezählt. Insgesamt wurde die Substanz an 25 verschiedenen Tumormodellen getestet. Zu den Tumormodellen gehörten Colon-, Pankreas- und Magenkarzinome, kleinzellige und nicht kleinzellige Lungentumore, Brust-, Ovar- und Nierenkarzinome sowie Melanome. Das breite Spektrum von verschiedenen Tumorentitäten sowie die große Anzahl von Modellen ermöglichen nach der Zellkultur eine erneute Prüfung der Wirksamkeit der Substanz und geben erste Hinweise darauf, ob die Substanz Wirkungsselektivität für bestimmte Tumorentitäten besitzt.

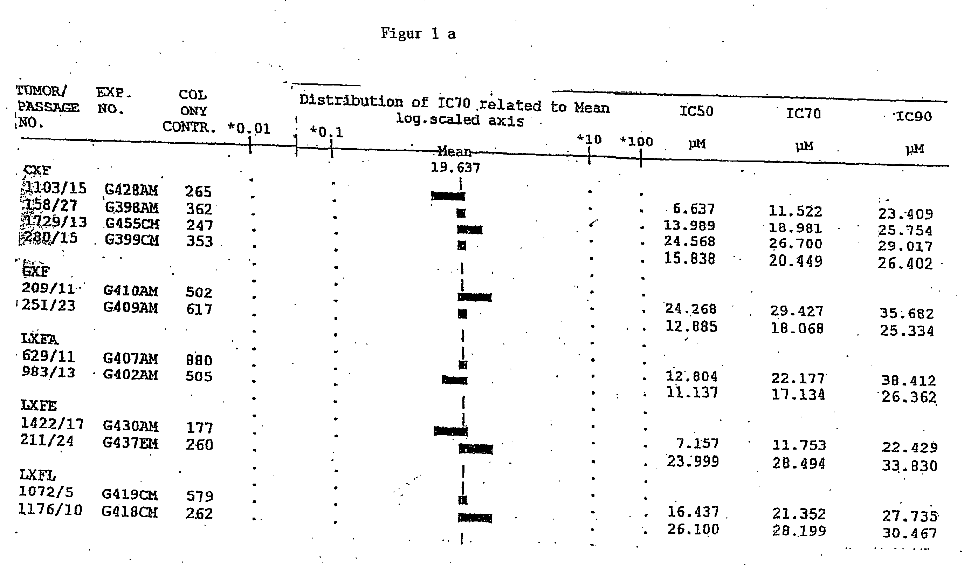

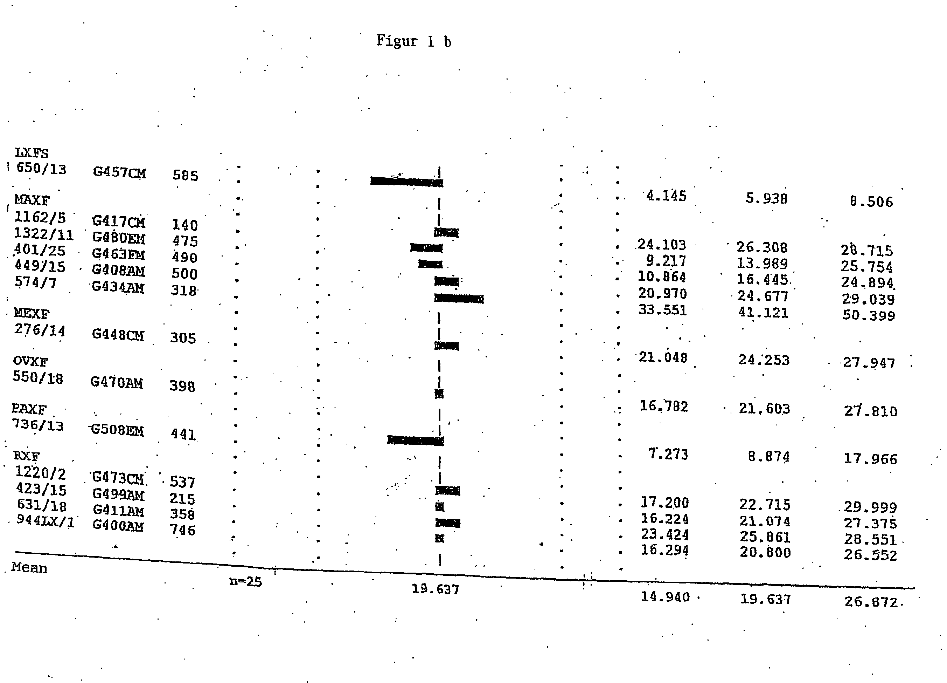

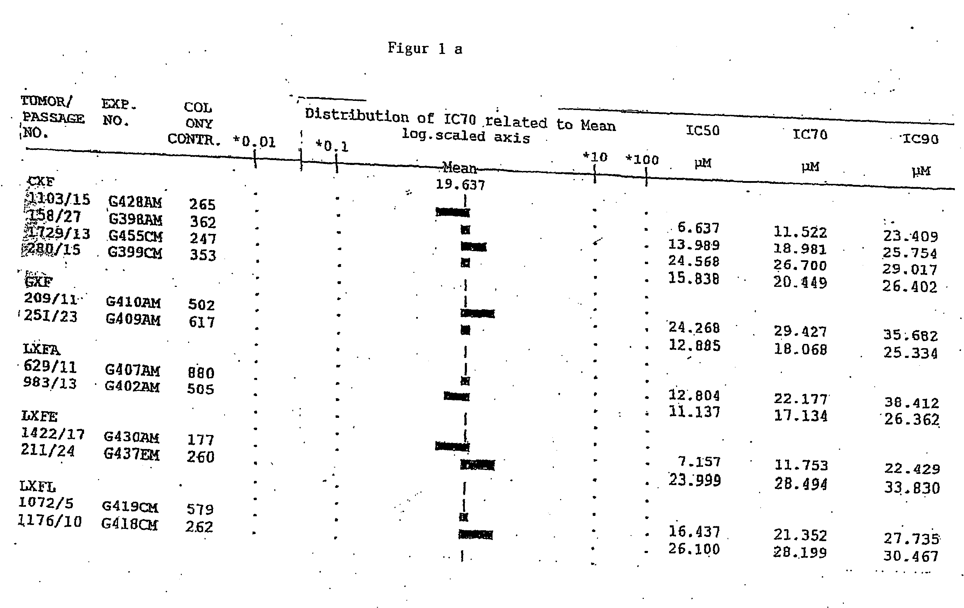

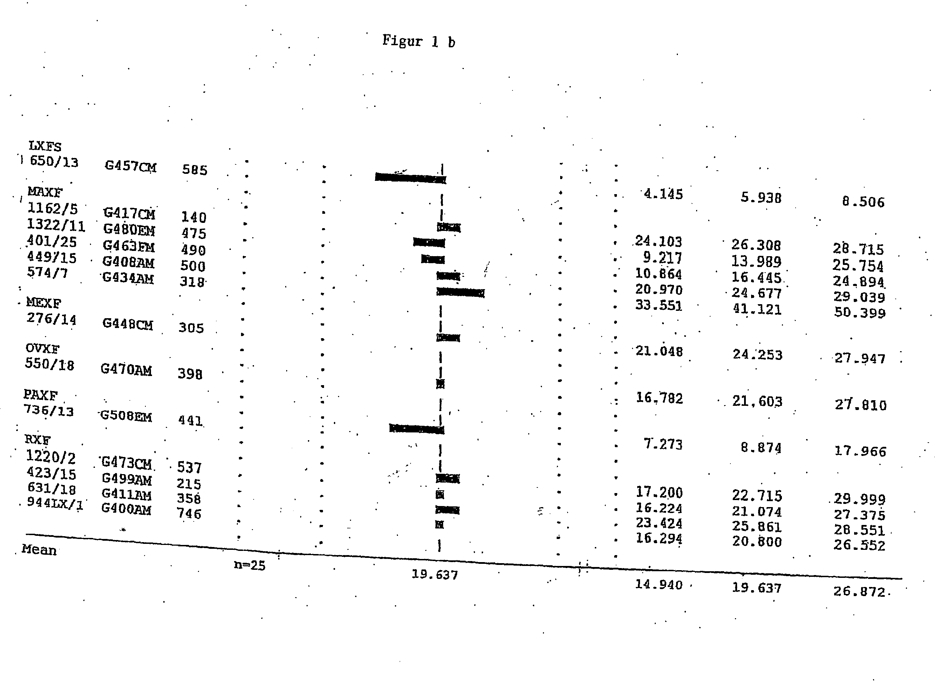

Die Ergebnisse sind in der Figur 1 dargestellt (graphische Analyse der Mittelwerte für die Substanz basierend auf den IC70-Werten). Die Balken stellen die IC70-Konzentrationen im Verhältnis zum Mittelwerte aller IC70-Werte dar. Bei Balken nach links ist der IC70-Wert für das betreffende Modell niedriger als der Mittelwert der IC70-Werte aller Modelle, d.h. diese Modelle sind sensitiver im Vergleich zum Durchschnitt aller Modelle. Balken nach rechts bedeuten höhere IC70-Werte als der Durchschnitt der Modelle und damit eine geringere Sensitivität.

Tumormodelle: CXF = Colonkarzinome, GXF = Magenkarzinome, LXFA = nicht kleinzellige Adenokarzinome der Lunge, LXFE = Plattenepithelkarzinome der Lunge, LXFL = großzellige Lungenkarzinome, LXFS = kleinzellige Lungenkarzinome, MAXF = Brusttumore, MEXF = Melanome, OVXF = Ovarkarzinome, PAXF = Pankreaskarzinome, RXF = Nierenkarzinome

Man erkennt, dass die Substanz im Kolonie-Assay mit sehr guten Ergebnissen getestet wurde. Die Substanz führte bei allen 25 Modellen zu einer Dosis-abhängigen Hemmung der Koloniebildung. Der mittlere IC50-Wert lag bei 15 µM. Der mittlere IC70-Wert betrug 20 µM. Die höchste Sensitivität zeigte ein nicht kleinzelliges Lungentumormodell mit einem IC50-Wert von 4 µM und einem IC70-Wert von 6 µM. Der höchste IC50-Wert (30 µM) bzw. IC70-Wert (40 µM) wurde für ein Brusttumormodell ermittelt.

Auch diese Ergebnisse belegen wiederum, dass die Substanz ein sehr breites Wirkspektrum zeigt. Es gab nicht ein einziges Modell, das nicht gehemmt wurde. Die Zellkulturergebnisse des Beispiels 3.1 ergaben bei den sechs getesteten Tumormodellen einen mittleren IC50-Wert von 6 µM und umspannten Werte zwischen 1 und 11 µM, so dass die Ergebnisse im Kolonie-Assay im Ergebnissbereich der Zellkulturversuche des Beispiels 3.1 liegen und diese folglich bestätigen.

Generell zeigen alle Kolonie-Assays, dass die Substanz vorteilhaft gegenüber dem Stand der Technik ist, weil sie ein breites therapeutisches Potential besitzt. Das breite Wirkspektrum spiegelt wider, dass die Substanz in Stoffwechselabläufe eingreift, die für alle Tumoren, unabhängig von der Entität, gemeinsam sind.

### 3.3: Vergleichsversuche

In einem Kolonie-Assay entsprechend dem Beispiel 3.2 wurden bei einem Ovarialkarzinom (OVXF 550) folgende Werte für das literaturbekannte SU11248, 5-[5-Fluoro-2-oxo-1,2-dihydroindol-(3Z)-ylidenemethyl]-2,4-dimethyl-1H-pyrrole-3-carboxylic Acid (2-Diethylaminoethyl)amide (PCT WO 01/60814 A2 und J. Med.Chem. 2003, 46, 1116-1119), gemessen:
OVXF 550: IC50 = 18,0µM, IC70 = 26,7µM

Im gleichen Kolonie-Assay und unter gleichen experimentellen Bedingungen wurden für die erfindungsgemäße Substanz (5-[5-Fluoro-2-oxo-1,2-dihydro-indol-(3Z)-ylidenemythyl]-2,4-dimethyl-1H-pyrrole-3-carboxylic acid (2-diethylamino-ethoxy)-amide (vorzugsweise das Salz der Äpfelsäure, Malate) folgende Werte gefunden:
OVXF 550: IC50 = 16,7 pM, IC70 = 21,6 µM

Man erkennt, dass die Werte für die erfindungsgemäße Substanz besser als für die bekannte Substanz sind.

Hierbei ist auch von wesentlicher Bedeutung, dass diese Vorteile auf den (einzigen) strukturellen Unterschied zurückzuführen sind, welcher in der erfindungsgemäßen -NH-O- Teilstruktur liegt. Hinzu kommt, dass bei erfindungsgemäßen Substanzen weniger Nebenwirkungen auf Grund dieser Teilstruktur zu erwarten sind.

## Patentansprüche

1. Verbindung gemäß Formel I 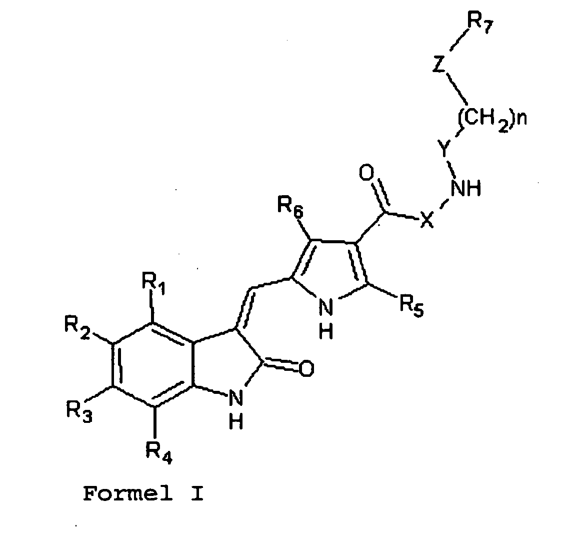 wobei R1 bis R4 gleich oder verschieden und -H, -F, -Cl, -Br, -I, oder (C₁-C₈)-Oxyalkyl sein können,
wobei R5 und R6 gleich oder verschieden und -H oder Cl-C4-Alkyl sein können,
wobei X für eine Bindung steht,
wobei Y und Z für eine chemische Bindung stehen können und wobei entweder Y oder Z für -O- steht,
wobei n = 0 bis 8 sein kann, und
wobei R7 eine -NR8R9 Gruppe,
in welcher R8 und R9 gleich oder verschieden und -H, eine (C₁-C₁₀)-Alkylgruppe oder (C₁-C₆)-Oxyalkyl sein können, eine (C₁-C₁₀)-Alkylgruppe oder eine gegebenenfalls teilweise oder vollständig halogenierte, insbesondere fluorierte, (C₁-C₁₀)-Alkylgruppe, (C₃-C₇)-Cycloalkylgruppe, (C₂-C₁₀)-Alkenylgruppe, (C₂-C₁₀) -Alkinylgruppe, (C₁-C₈)-Alkyl(C₃-C₇)cycloalkylgruppe, (C₂-C₈)-Alkenyl(C₃-C₇)cycloalkylgruppe, Heterocyclylgruppe, (C₁-C₈)-Alkylheterocyclylgruppe, (C₂-C₈)-Alkenylheterocyclylgruppe, Arylgruppe, (C₁-C₉)-Alkylarylgruppe, (C₂-C₈)-Alkenylarylgruppe, oder (C₂-C₈)-Alkinylarylgruppe, oder eine gegebenenfalls durch 1-2 Ketogruppen, 1-2 (C₁-C₅)-Alkylgruppen, 1-2 (C₁-C₅)-Alkoxygruppen, 1-3 Halogenatome, 1-2 Exomethylengruppen substituierte, 1-3 Stickstoffatome und/oder 1-2-Sauerstoffatome und/oder 1-2 Schwefelatome enthaltende mono- oder bizyklische Heteroarylgruppe, eine (C₁-C₈)-Alkylheteroarylgruppe, oder eine (C₂-C₈)-Alkenylheteroarylgruppe, eine (C₂-C₈)-Alkinylheteroarylgruppe ist, wobei diese Gruppen über eine beliebige Position mit R2 verknüpft sein können und gegebenenfalls an einer oder mehreren Stellen hydriert sein können,
und Stereoisomere sowie physiologisch verträgliche Salze solcher Verbindungen.

2. Verbindung nach Anspruch 1, wobei R1, R3 und R4 -H sind und wobei R2 -F, -Cl, -Br, -I, oder (C₁-C₈)-Oxyalkyl ist.

3. Verbindung nach Anspruch 1 oder 2, wobei R5 und R6 (C₁-C₃)-Alkyl, insbesondere Methyl, sind.

4. Verbindung nach einem der Ansprüche 1 bis 3, wobei n = 1 oder 2 ist.

5. Verbindung nach einem der Ansprüche 1 bis 4, wobei R7 eine -NR8R9 Gruppe, in welcher R8 und R9 gleich oder verschieden und -H, Methyl, Ethyl, Methoxy oder Ethoxy sind, ist, oder wobei R7 pyrrolidin-1-yl, morpholin-4-yl, pyridin-4-yl, 1-methyl-4-piperidyl, oder triazol-1-yl, optional mit (C₁-C₆)-Alkyl, -F, -Cl, -Br, -I, oder (C₁-C₆)-Oxyalkyl einfach oder mehrfach substituiert, ist.

6. Verbindung nach einem der Ansprüche 1 bis 5, nämlich
5-[2-Oxo-1,2-dihydro-indol-(3Z)-ylidenemethyl]-2,4-dimethyl-1H-pyrrole-3-carboxylic acid (2-diethylamino-ethoxy)-amide,
5-[5-Fluoro-2-oxo-1,2-dihydro-indol-(3Z)-ylidenemethyl]-2,4-dimethyl-1H-pyrrole-3-carboxylic acid (2-diethylamino-ethoxy)-amide,
5-[5-Bromo-2-oxo-1,2-dihydro-indol-(3Z)-ylidenemethyl]-2,4-dimethyl-1H-pyrrole-3-carboxylic acid (2-diethylamino-ethoxy)-amide,
5-[5-Chloro-2-oxo-1,2-dihydro-indol-(3Z)-ylidenemethyl]-2,4-dimethyl-1H-pyrrole-3-carboxylic acid (2-diethylamino-ethoxy)-amide,
5-[6-Fluoro-2-oxo-1,2-dihydro-indol-(3Z)-ylidenemethyl]-2,4-dimethyl-1H-pyrrole-3-carboxylic acid (2-diethylamino-ethoxy)-amide,
5-[6-Chloro-2-oxo-1,2-dihydro-indol-(3Z)-ylidenemethyl]-2,4-dimethyl-1H-pyrrole-3-carboxylic acid (2-diethylamino-ethoxy)-amide,
5-[6-Bromo-2-oxo-1,2-dihydro-indol-(3Z)-ylidenemethyl]-2,4-dimethyl-1H-pyrrole-3-carboxylic acid (2-diethylamino-ethoxy)-amide,
5-[5-Methyl-2-oxo-1,2-dihydro-indol-(3Z)-ylidenemethyl]-2,4-dimethyl-1H-pyrrole-3-carboxylic acid (2-diethylamino-ethoxy)-amide,
5-[5-Methoxy-2-oxo-1,2-dihydro-indol-(3Z)-ylidenemethyl]-2,4-dimethyl-1H-pyrrole-3-carboxylic acid (2-diethylamino-ethoxy)-amide,
5-[6-Methyl-2-oxo-1,2-dihydro-indol-(3Z)-ylidenemethyl]-2,4-dimethyl-1H-pyrrole-3-carboxylic acid (2-diethylamino-ethoxy)-amide,
oder Derivate der vorstehenden Verbindungen, wobei die 2-diethylamino-ethoxy-Gruppe ersetzt ist durch eine der Gruppen ausgewählt aus "2-(triazol-1-yl)ethoxy, 2-triazol-1-yl)methoxy, (pyridin-4-yl)methoxy, 2-(pyridiny-4-yl)ethoxy, 2-(morpholin-4-yl)ethoxy, (morpholin-4-yl)methoxy, (1-methyl-4-piperidyl)methoxy, 2-(1-methyl-4-piperidyl)ethoxy, 2-(pyrrolidin-1-yl)ethoxy, (pyrrolidin-1-yl)methoxy, 2-(dimethylamino)ethoxy, (dimethylamino)methoxy und (dimethylamino)methoxy".

7. Pharmazeutische Zusammensetzung enthaltend eine physiologisch wirksame Dosis einer Verbindung nach einem der Ansprüche 1 bis 6 sowie optional physiologisch verträgliche Hilfs- und/oder Trägerstoffe.

8. Pharmazeutische Zusammensetzung nach Anspruch 7 zusätzlich enthaltend eine physiologisch wirksame Dosis eines von der Verbindung verschiedenen Wirkstoffes, insbesondere ausgewählt aus der Gruppe bestehend aus "Aldesleukin, Amifostine, Atrasentan, Bevacizumab, Bexaroten, Bortezomib, Capecitabine, Carboplatin, Chlorambucil, Cisplatin, Cladribine, Cyclophosphamid, Cytamid, Dacarbazin, Docetaxel, Droloxifene, Edrecolomab, Epothilone, Erlotinib, Etoposide, Exemestane, Flavopiridol, Fludarabine, Fuorouracil, Formestane, Fulvestrant, Gefitinib, Gemcitabine, Idarubicin, Irinotecan, Ixabepilone, Lonafarnib, Miltefosine, Mitomycin, Neovastat, Oxaliplatin, Pemetrexed, Porfimer, Rituximab, Tegafur, Temozolomide, Tipifarnib, Topotecan, Trimetrexate, Vorozole, Vinblastine, und Mischungen von zwei oder mehreren solcher wirkstoffe".

9. Verwendung einer Verbindung nach einem der Ansprüche 1 bis 6, optional in Mischung mit einem Wirkstoff oder mehreren verschiedenen Wirkstoffen nach Anspruch 8, zur Herstellung einer pharmazeutischen Zusammensetzung zur Behandlung einer proliferativen oder inflammatorischen Erkrankung einhergeht, welche ausgewählt ist aus der Gruppe bestehend aus "Krebs, wie Lungenkrebs, Leukämie, Eierstockkrebs, Sarkome, Meningiom, Darmkrebs, Lyphknotenkrebs, Hirntumore, Brustkrebs, Pankreaskrebs, Prostatakrebs, Hautkrebs, chronische Entzündungen, Asthma, Allergie, Rhinitis, Uveitis, Urticaria, Arthritis, Osteaoarthritis, chronische Polyarthritis, rheumatische Arthritis, Inflammatory bowl disease, degenerative Gelenkserkrankungen, Erkrankungen des rheumatischen Formenkreises mit Knorpelabbau, Sepsis, Autoimmunerkrankungen, Typ I Diabetes, Hashimoto-Thyreoiditis, Autoimmunthrombozytopenie, Multiple Sklerose, Myasthenia gravis, chronisch entzündliche Darmerkrankungen, Morbus Crohn, Uveitis, Psoriasis, atypische Dermatitits, Kollagenosen, Goodpasture-Syndrom, Erkrankungen mit gestörter Leukozyten-Adhäsion, Cachexie, Erkrankungen durch erhöhte TNFalpha Konzentration, Diabetes, Adipositas, bakterielle Infektionen, insbesondere mit resistenten Bakterien, Herzinsuffizienz und der Chronic Cardiac Failure (CCF)".

10. Verwendung nach Anspruch 9, wobei eine Verbindung nach einem der Ansprüche 1 bis 6 in physiologisch wirksamer Dosis mit zumindest einem Träger und/oder Hilfsstoff gemischt und in eine galenische Darreichungsform gebracht wird.

11. Verfahren zur Herstellung einer Verbindung nach einem der Ansprüche 1 bis 6, wobei eine Substanz der Formel II 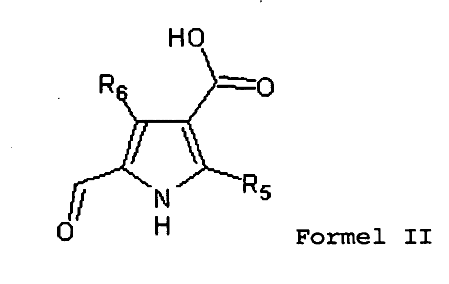 mit einer Substanz der Formel III 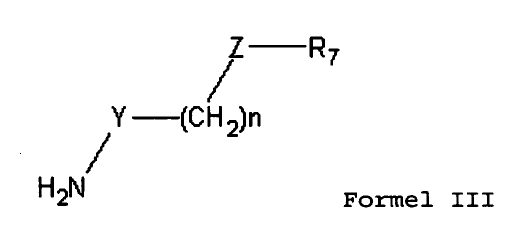 zu einer Substanz der Formel IV 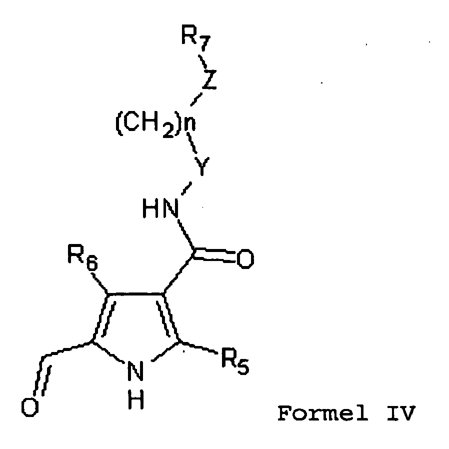 umgesetzt wird, wobei dann die Substanz der Formel IV mit einer Substanz der Formel V 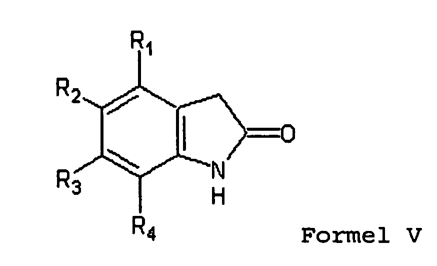 zu einer Verbindung der Formel VI 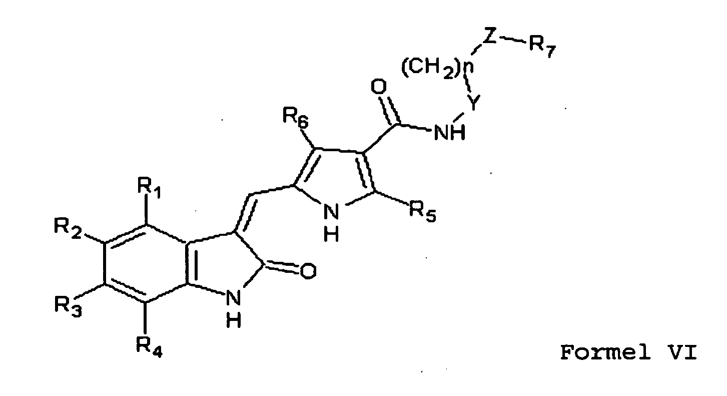 umgesetzt wird, wobei die Reste R1 bis R7 sowie die Gruppen Y und Z die Bedeutungen der Ansprüche 1 bis 6 haben.

## Claims

1. A compound according to Formula I 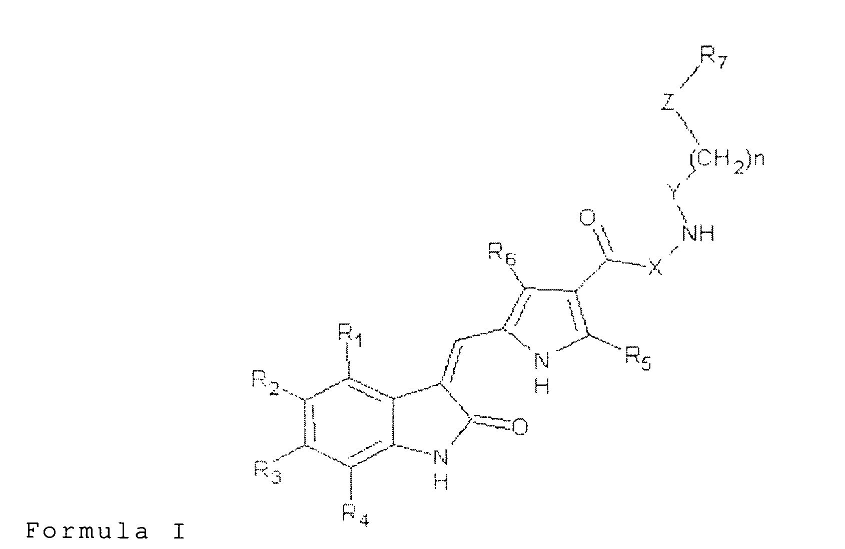 wherein R1 to R4 may be identical or different and may be -H, -F, -Cl, -Br, -I or (C₁-C₈)oxyalkyl,
wherein R5 and R6 may be identical or different and may be -H or C1-C4 alkyl,
wherein X is a binding,
wherein Y and Z may be a chemical binding and wherein either Y or Z is -O-,
wherein n may be 0 to 8, and
wherein R7 may be an -NR8R9 group, in which R8 and R9 may be identical or different and may be -H, a (C₁-C₁₀) alkyl group or (C₁-C₆) oxyalkyl, a (C₁-C₁₀) alkyl group or, if applicable, a partially or completely halogenated, in particular fluorinated (C₁-C₁₀) alkyl group, (C₃-C₇) cycloalkyl group, (C₂-C₁₀) alkenyl group, (C₂-C₁₀) alkinyl group, (C₁-C₈) alkyl-(C₃-C₇) cycloalkyl group, (C₂-C₈) alkenyl-(C₃-C₇) cycloalkyl group, heterocyclyl group, (C₁-C₈) alkylheterocyclyl group, (C₂-C₈) alkenylheterocyclyl group, aryl group, (C₁-C₈) alkylaryl group, (C₂-C₈) alkenylaryl group, or (C₂-C₈) alkinylaryl group, or a monocyclic or bicyclic heteroaryl group, if applicable, substituted by 1-2 keto groups, 1-2 (C₁-C₅) alkyl groups, 1-2 (C₁-C₅) alkoxy groups, 1-3 halogen atoms, 1-2 exo-methyl groups, and containing 1-3 nitrogen atoms and/or 1-2 oxygen atoms and/or 1-2 sulfur atoms, a (C₁-C₈) alkylheteroaryl group, or a (C₂-C₈) alkenylheteroaryl group, a (C₂-C₈) alkinylheteroaryl group, wherein these groups may be linked at an arbitrary position with R2 and, if applicable, may be hydrated at one or several positions,
and stereoisomers and physiologically well-tolerated salts of such compounds.

2. The compound according to claim 1, wherein R1, R3 and R4 are -H, and wherein R2 is -F, -Cl, -Br, -I or (C₁-C₈) oxyalkyl.

3. The compound according to claim 1 or 2, wherein R5 and R6 are (C₁-C₃) alkyl, in particular methyl.

4. The compound according to one of claims 1 to 3, wherein n is 1 or 2.

5. The compound according to one of claims 1 to 4, wherein R7 is an -NR8R9 group, in which R8 and R9 are identical or different and are -H, methyl, ethyl, methoxy or ethoxy, or wherein R7 is pyrrolidine-1-yl, morpholine-4-yl, pyrridine-4-yl, 1-methyl-4-piperidyl, or triazole-1-yl, optionally substituted singly or multiply with (C₁-C₆) alkyl, -F, -Cl, -Br, -I, or (C₁-C₆) oxyalkyl.

6. The compound according to one of claims 1 to 5, namely
5-[2-oxo-1,2-dihydro-indole-(3Z)-ylidenemethyl]-2,4-dimethyl-1H-pyrrole-3-carboxylic acid (2-di-ethylaminoethoxy)-amide,
5-[5-fluoro-2-oxo-1,2-dihydro-indole-(3Z)-ylidene-methyl]-2,4-dimethyl-1H-pyrrole-3-carboxylic acid (2-diethylamino-ethoxy)-amide,
5-[5-bromo-2-oxo-1,2-dihydro-indole-(3Z)-ylidene-methyl]-2,4-dimethyl-1H-pyrrole-3-carboxylic acid (2-diethylamino-ethoxy)-amide,
5-[5-chloro-2-oxo-1,2-dihydro-indole-(3Z)-ylidene-methyl]-2,4-dimethyl-1H-pyrrole-3-carboxylic acid (2-diethylamino-ethoxy)-amide,
5-[6-fluoro-2-oxo-1,2-dihydro-indole-(3Z)-ylidene-methyl]-2,4-dimethyl-1H-pyrrole-3-carboxylic acid (2-diethylamino-ethoxy)-amide,
5-[6-chloro-2-oxo-1,2-dihydro-indole-(3Z)-ylidene-methyl]-2,4-dimethyl-1H-pyrrole-3-carboxylic acid (2-diethylamino-ethoxy)-amide,
5-[6-bromo-2-oxo-1,2-dihydro-indole-(3Z)-ylidene-methyl]-2,4-dimethyl-1H-pyrrole-3-carboxylic acid (2-diethylamino-ethoxy)-amide,
5-[5-methyl-2-oxo-1,2-dihydro-indole-(3Z)-ylidene-methyl]-2,4-dimethyl-1H-pyrrole-3-carboxylic acid (2-diethylamino-ethoxy)-amide,
5-[5-methoxy-2-oxo-1,2-dihydro-indole-(3Z)-ylid-enemethyl]-2,4-dimethyl-1H-pyrrole-3-carboxylic acid (2-diethylamino-ethoxy)-amide,
5-[6-methyl-2-oxo-1,2-dihydro-indole-(3Z)-ylidene-methyl]-2,4-dimethyl-1H-pyrrole-3-carboxylic acid (2-diethylamino-ethoxy)-amide,
or derivatives of the above compounds, wherein the 2-diethylamino-ethoxy group is replaced by one of the groups selected from "2-(triazole-1-yl) ethoxy, 2-triazole-1-yl) methoxy, (pyridine-4-yl) methoxy, 2-(pyridine-4-yl) ethoxy, 2-(morpholine-4-yl) ethoxy, (morpholine-4-yl) methoxy, (1-methyl-4-piperidyl) methoxy, 2-(1-methyl-4-piperidyl) ethoxy, 2-(pyrrolidine-1-yl) ethoxy, (pyrrolidine-1-yl) methoxy, 2-(dimethylamino) ethoxy, (dimethylamino) methoxy and (dimethylamino) methoxy".

7. A pharmaceutical composition containing a physiologically effective dose of a compound according to one of claims 1 to 6 and optionally physiologically well tolerated auxiliary and/or carrier substances.

8. The pharmaceutical composition according to claim 7, additionally containing an active substance being different from the compound, in particular selected from the group consisting of "aldesleukin, amifostine, atrasentan, bevacizumab, bexaroten, bortezomib, capecitabine, carboplatin, chlorambucil, cisplatin, cladribine, cyclophosphamid, cytamid, dacarbazin, docetaxel, droloxifene, edrecolomab, epothilone, erlotinib, etoposide, exemestane, flavopiridol, fluorouracil, formestane, fulvestrant, gefitinib, gemcitabine, idarubicin, irinotecan, ixabepilone, lonafarnib, miltefosine, mitomycin, neovastat, oxaliplatin, pemetrexed, porfimer, rituximab, tegafur, temozolomide, tipifarnib, topotecan, trimetrexate, vorozole, vinblastine, and mixtures of two or more of such active substances".

9. The use of a compound according to one of claims 1 to 6, optionally in a mixture with an active substance or several different active substances according to claim 8, for producing a pharmaceutical composition for the prophylaxis or treatment of a proliferative or inflammatory disease, which is selected from the group consisting of "cancer, such as lung cancer, leukemia, ovarian cancer, sarcoma, meningioma, intestine cancer, lymph node cancer, brain tumors, breast cancer, pancreas cancer, prostate cancer, skin cancer, chronic inflammations, asthma, allergy, rhinitis, uveitis, urticaria, arthritis, osteoarthritis, chronic polyarthritis, rheumatoid arthritis, inflammatory bowel disease, degenerative joint diseases, diseases of the rheumatic form circle with cartilage breakdown, sepsis, autoimmune diseases, type I diabetes, Hashimoto thyreoiditis, autoimmune thrombocytopenia, multiple sclerosis, myasthenia gravis, chronic inflammatory intestinal diseases, Crohn's disease, uveitis, psoriasis, atypical dermatitis, collagenoses, Goodpasture syndroma, diseases with disturbed leukocyte adhesion, cachexia, diseases by increased TNF-alpha concentration, diabetes, adipositas, bacterial infections, in particular with resistant bacteria, heart insufficiency and the chronic cardiac failure (CCF)".

10. The use according to claim 9, wherein a compound according to one of claims 1 to 6 is mixed in a physiologically effective dose with at least one carrier and/or auxiliary substance and brought into a galenic form of administration.

11. A method for producing a compound according to one of claims 1 to 6, wherein a substance of Formula II 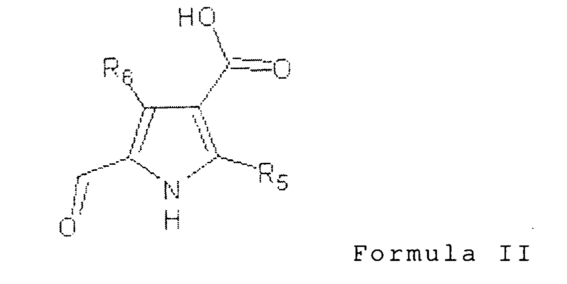 is reacted with a substance of Formula III 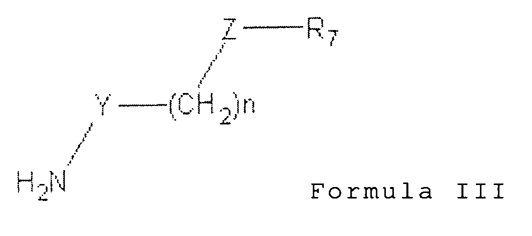 to a substance of Formula IV 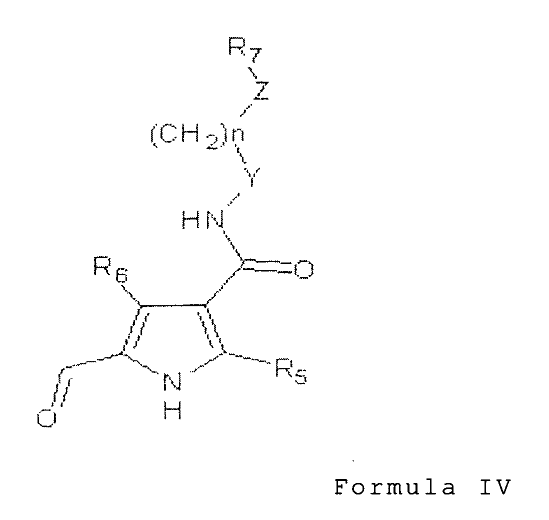 wherein then the substance of Formula IV is reacted with a substance of Formula V 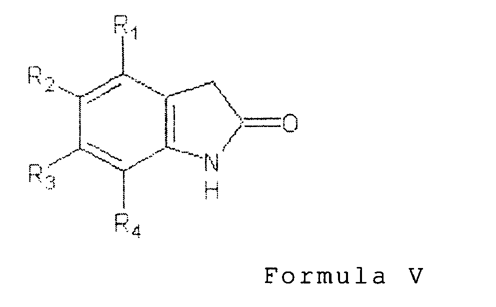 to a compound of Formula VI 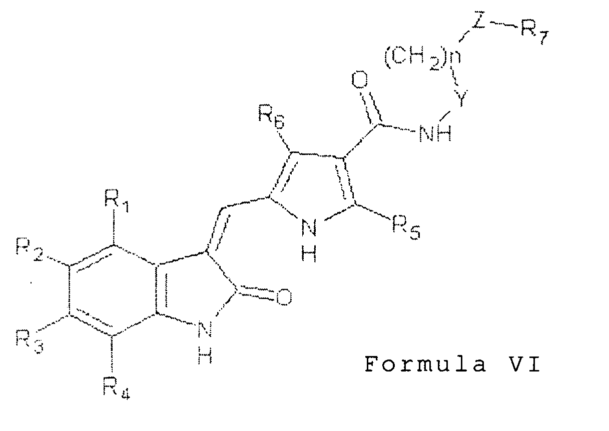 wherein the radicals R1 to R7 and the groups Y and Z have the meanings of claims 1 to 6.

## Revendications

1. Composé selon la Formule I 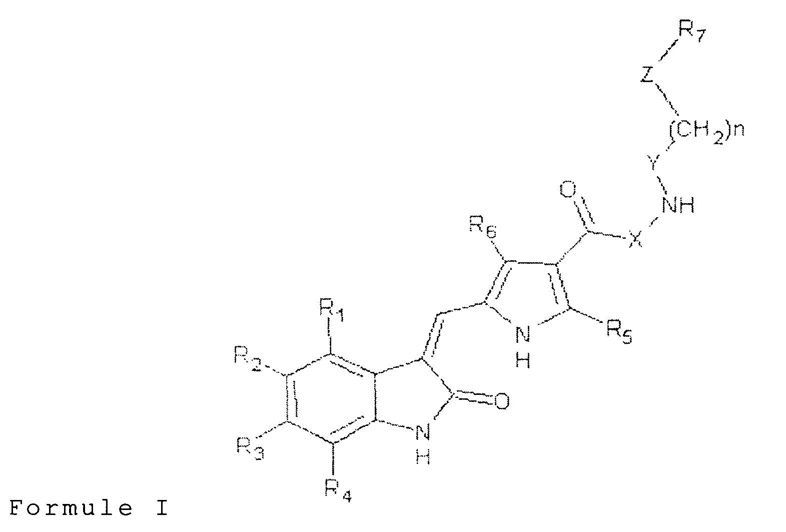 dans lequel R1 à R4 peuvent être identiques ou différents et peuvent être -H, -F, -Cl, -Br, -I ou (C₁-C₈) oxyalkyle,
dans lequel R5 et R6 peuvent être identiques ou différents et peuvent être -H ou C1-C4 alkyle,
dans lequel X est un liage,
dans lequel Y et Z peuvent être un liage chimique et dans lequel Y ou Z est -O-,
dans lequel n peut être 0 à 8, et
dans lequel R7 peut être un groupe -NR8R9, dans lequel R8 et R9 peuvent être identiques ou différents et peuvent être -H, un groupe (C₁-C₁₀) alkyle ou (C₁-C₆) oxyalkyle, un groupe (C₁-C₁₀) alkyle ou un groupe (C₁-C₁₀) alkyle, le cas échéant, partiellement ou complètement halogéné, en particulier fluoré, un groupe (C₃-C₇) cycloalkyle, un groupe (C₂-C₁₀) alcényle, un groupe (C₂-C₁₀) alcynyle, un groupe (C₁-C₈) alkyle-(C₃-C₇) cycloalkyle, un groupe (C₂-C₈) alcényle-(C₃-C₇) cycloalkyle, un groupe hétérocyclyle, un groupe (C₁-C₈) alkyle hétérocyclyle, un groupe (C₂-C₈) alcényle hétérocyclyle, un groupe aryle, un groupe (C₁-C₈) alkyle aryle, un groupe (C₂-C₈) alcényle aryle, ou un groupe (C₂-C₈) alcynyle aryle, partiellement ou un groupe hétéroaryle monocyclique ou bicyclique, le cas échéant, substitué par 1-2 groupes cétones, 1-2 groupes (C₁-C₅) alkyle, 1-2 groupes (C₁-C₅) alcoxy, 1-3 atomes d'halogène, 1-2 groupes d'exométhyle, et contenant 1-3 atomes de nitrogène et/ou 1-2 atomes d'oxygène et/ou 1-2 atomes de soufre, un groupe (C₁-C₈) alkyle hétéroaryle, ou un groupe (C₂-C₈) alcényle hétéroaryle, un groupe (C₂-C₈) alcynyle hétéroaryle, dans lequel ces groupes peuvent être liés à une position arbitraire avec R2 et, le cas échéant, peuvent être hydratés à une ou plusieurs positions,
et des stéréoisomères et sels physiologiquement bien tolérés de tels composés.

2. Composé selon la revendication 1, dans lequel R1, R3 et R4 sont -H, et dans lequel R2 est -F, - Cl, -Br, -I ou (C₁-C₈) oxyalkyle.

3. Composé selon la revendication 1 ou 2, dans lequel R5 et R6 sont (C₁-C₃) alkyle, en particulier méthyle.

4. Composé selon une des revendications 1 à 3, dans lequel n est 1 ou 2.

5. Composé selon une des revendications 1 à 4, dans lequel R7 est un groupe -NR8R9, dans lequel R8 et R9 sont identiques ou différents et sont -H, méthyle, éthyle, méthoxy ou éthoxy, ou dans lequel R7 est pyrrolidine-1-yle, morpholine-4-yle, pyrridine-4-yle, 1-méthyle-4-pipéridyle, ou triazole-1-yle, optionnellement substitué par un ou plusieurs de (C₁-C₆) alkyle, -F, -Cl, -Br, -I, ou (C₁-C₆) oxyalkyle.

6. Composé selon une des revendications 1 à 5, c'est-à-dire
5-[2-oxo-1,2-dihydro-indole-(3Z)-ylidèneméthyle]-2,4-diméthyle-1H-pyrrole-3-acide carboxylique (2-diéthyleaminoéthoxy)-amide,
5-[5-fluoro-2-oxo-1,2-dihydro-indole-(3Z)-ylidène-méthyle]-2,4-diméthyle-1H-pyrrole-3-acide carboxylique (2-diéthyleamino-éthoxy)-amide,
5-[5-bromo-2-oxo-1,2-dihydro-indole-(3Z)-ylidène-méthyle]-2,4-diméthyle-1H-pyrrole-3-acide carboxylique (2-diéthyleamino-éthoxy)-amide,
5-[5-chloro-2-oxo-1,2-dihydro-indole-(3Z)-ylidène-méthyle]-2,4-diméthyle-1H-pyrrole-3-acide carboxylique (2-diéthyleamino-éthoxy)-amide,
5-[6-fluoro-2-oxo-1,2-dihydro-indole-(3Z)-ylidène-méthyle]-2,4-diméthyle-1H-pyrrole-3-acide carboxylique (2-diéthyleamino-éthoxy)-amide,
5-[6-chloro-2-oxo-1,2-dihydro-indole-(3Z)-ylidène-méthyle]-2,4-diméthyle-1H-pyrrole-3-acide carboxylique (2-diéthyleamino-éthoxy)-amide,
5-[6-bromo-2-oxo-1,2-dihydro-indole-(3Z)-ylidène-méthyle]-2,4-diméthyle-1H-pyrrole-3-acide carboxylique (2-diéthyleamino-éthoxy)-amide,
5-[5-méthyle-2-oxo-1,2-dihydro-indole-(3Z)-ylidè-neméthyle]-2,4-diméthyle-1H-pyrrole-3-acide carboxylique (2-diéthyleamino-éthoxy)-amide,
5-[5-méthoxy-2-oxo-1,2-dihydro-indole-(3Z)-ylidè-neméthyle]-2,4-diméthyle-1H-pyrrole-3-acide carboxylique (2-diéthyleamino-éthoxy)-amide,
5-[6-méthyle-2-oxo-1,2-dihydro-indole-(3Z)-ylidè-neméthyle]-2,4-diméthyle-1H-pyrrole-3-acide carboxylique (2-diéthyleamino-éthoxy)-amide,
ou des dérivés des composés ci-dessus, dans lequel le groupe 2-diéthyleamino-éthoxy est remplacé par un des groupes choisis à partir de "2-(triazole-1-yle) éthoxy, 2-triazole-1-yle) méthoxy, (pyridine-4-yle) méthoxy, 2-(pyridine-4-yle) éthoxy, 2-(morpholine-4-yle) éthoxy, (morpholine-4-yle) méthoxy, (1-méthyle-4-pipéridyle) méthoxy, 2-(1-méthyle-4-pipéridyle) éthoxy, 2-(pyrrolidine-1-yle) éthoxy, (pyrrolidine-1-yle) méthoxy, 2-(diméthyleamino) éthoxy, (diméthyleamino) méthoxy et (diméthyleamino) méthoxy".

7. Composition pharmaceutique comprenant une dose physiologiquement efficace d'un composé selon une des revendications 1 à 6 et optionnellement des substances auxiliaires et/ou porteuses physiologiquement bien tolérées.

8. Composition pharmaceutique selon la revendication 7, en addition comprenant une substance active étant différente du composé, en particulier choisie à partir du groupe comprenant «aldesleukine, amifostine, atrasentan, bevacizumab, bexarotène, bortezomib, capécitabine, carboplatine, chlorambucil, cisplatine, cladribine, cyclophosphamide, cytamide, dacarbazine, docétaxel, droloxifène, édrécolomab, épothilone, erlotinib, étoposide, exémestane, flavopiridol, fluorouracil, formestane, fulvestrant, gefitinib, gemcitabine, idarubicine, irinotécan, ixabepilone, lonafarnib, miltéfosine, mitomycine, neovastat, oxaliplatine, pemetrexed, porfimer, rituximab, tégafur, témozolomide, tipifarnib, topotécan, trimétrexate, vorozole, vinblastine, et des mélanges de deux ou plus de telles substances actives».

9. Utilisation d'un composé selon une des revendications 1 à 6, optionnellement dans un mélange avec une substance active ou plusieurs substances actives différentes selon la revendication 8, pour produire une composition pharmaceutique pour la prophylaxie ou le traitement d'une maladie proliférative ou inflammatoire, qui est choisie à partir du groupe comprenant «cancer, comme p.ex. cancer du poumon, leucémie, cancer de l'ovaire, sarcome, méningiome, cancer du côlon, cancer des noeuds lymphatiques, tumeurs du cerveau, cancer du sein, cancer du pancréas, cancer de la prostate, cancer de la peau, inflammations chroniques, asthme, allergie, rhinite, uvéite, urticaire, arthrite, ostéoarthrite, polyarthrite chronique, arthrite rhumatoïde, maladie inflammatoire de l'intestin, maladie d'articulaire dégénérative, maladies rhumatismales avec dégradation du cartilage, septicémie, maladies auto-immunes, diabète type I, thyroïdite de Hashimoto, thrombocytopénie auto-immune, sclérose multiple, myasthénie grave, maladies de l'intestin inflammatoires chroniques, maladie de Crohn, uvéite, psoriasis, dermatite atypique, collagénoses, syndrome de Goodpasture, maladies avec des dysfonctions de l'adhésion de leucocytes, cachexie, maladies par concentration de TNF-alpha augmentée, diabète, obésité, infections bactériennes, en particulier avec des bactéries résistantes, insuffisance cardiaque et défaillance cardiaque chronique (CCF)».

10. Utilisation selon la revendication 9, dans laquelle un composé selon une des revendications 1 à 6 est mélangé dans une dose physiologiquement efficace avec au moins une substance porteuse et/ou auxiliaire et transformé en une forme galénique d'administration.

11. Procédé de production d'un composé selon une des revendications 1 à 6, dans lequel une substance de la Formule II 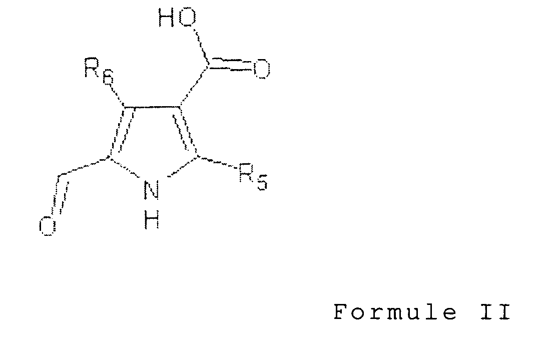 est réagie avec une substance de la Formule III 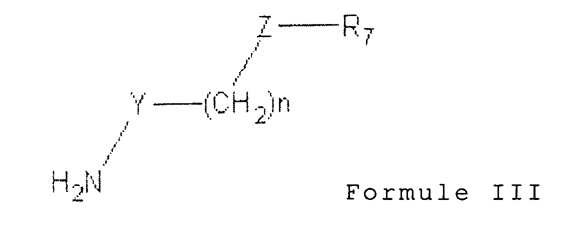 à une substance de la Formule IV 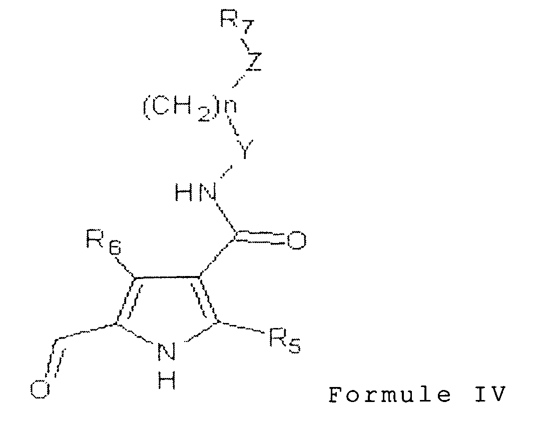 dans lequel puis la substance de la Formule IV est réagie avec une substance de la Formule V 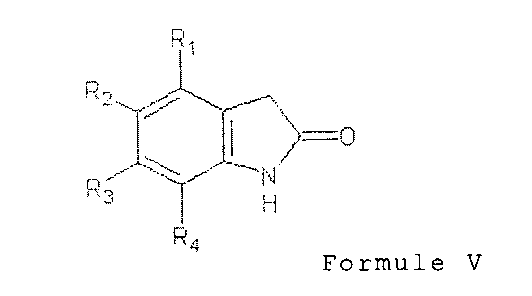 à un composé de la Formule VI 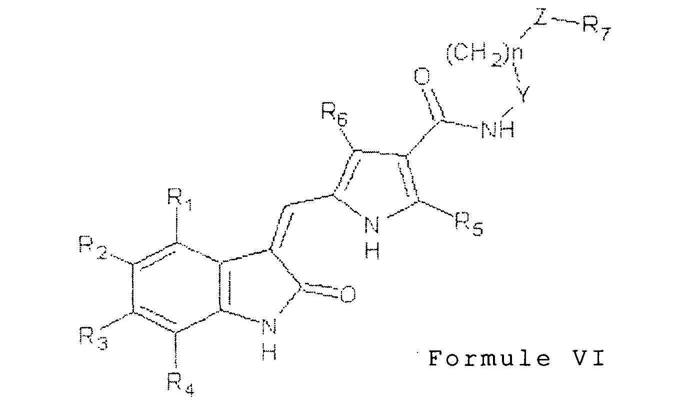 dans lequel les radicaux R1 à R7 et les groupes Y et Z ont les sens des revendications 1 à 6.
